# EUROPEAN PATENT SPECIFICATION

(11) **EP 2 190 989 B1**
(45) Date of publication and mention of the grant of the patent: **21.05.2014**
(21) Application number: 08803361.8
(22) Date of filing: 29.08.2008
(51) Int. Cl.: C12N 15/10, C07K 14/31, C07K 14/47

(54) **METHOD FOR MANUFACTURING A MODIFIED PEPTIDE**
VERFAHREN ZUR HERSTELLUNG EINES MODIFIZIERTEN PEPTIDS
PROCÉDÉ DE FABRICATION D'UN PEPTIDE MODIFIÉ

(30) Priority: 30.08.2007 EP 07450150
(43) Date of publication of application: 02.06.2010
(73) Proprietor: Procomcure Biotech GmbH, 4020 Linz (AT)
(72) Inventor: ÖNDER, Kamil, A-5020 Salzburg (AT); BAUER, Johann W., A-5020 Salzburg (AT)
(74) Representative: Sonn & Partner Patentanwälte
(86) International application number: PCT/EP2008/061362
(87) International publication number: WO 2009/027495

(56) References cited:
- EP-A- 1 405 911
- WO-A-00/66722
- WO-A-02/31165
- US-A1- 2003 186 385
- WILLIAMS THOMAS M ET AL: "Group 13 HOX proteins interact with the MH2 domain of R-Smads and modulate Smad transcriptional activation functions independent of HOX DNA-binding capability." NUCLEIC ACIDS RESEARCH 2005, vol. 33, no. 14, 2005, pages 4475-4484, XP002471419 ISSN: 1362-4962
- YAMAGUCHI Y ET AL: "A novel Cdc42-interacting domain of the yeast polarity establishment protein Bem1: Implications for modulation of mating pheromone signaling" JOURNAL OF BIOLOGICAL CHEMISTRY 05 JAN 2007 UNITED STATES, vol. 282, no. 1, 5 January 2007 (2007-01-05), pages 29-38, XP002471420 ISSN: 0021-9258 1083-351X -& DREES BECKY L ET AL: "A protein interaction map for cell polarity development" JOURNAL OF CELL BIOLOGY, vol. 154, no. 3, 6 August 2001 (2001-08-06), pages 549-571, XP002471422 ISSN: 0021-9525
- ALLEN J B ET AL: "Finding prospective partners in the library: the two-hybrid system and phage display find a match" TRENDS IN BIOCHEMICAL SCIENCES, ELSEVIER, HAYWARDS, GB, vol. 20, no. 12, December 1995 (1995-12), pages 511-516, XP004222355 ISSN: 0968-0004
- PAJUNEN MARIA ET AL: "High-precision mapping of protein-protein interfaces: an integrated genetic strategy combining en masse mutagenesis and DNA-level parallel analysis on a yeast two-hybrid platform" NUCLEIC ACIDS RESEARCH, vol. 35, no. 16, August 2007 (2007-08), page Article No.: e103, XP002471421 ISSN: 0305-1048
- WRENN S JARRETT ET AL: "Chemical evolution as a tool for molecular discovery", ANNUAL REVIEW OF BIOCHEMISTRY, vol. 76, 2007, pages 331-349, ISSN: 0066-4154
- PATTANAIK S ET AL: "Directed evolution of plant basic helix-loop-helix transcription factors for the improvement of transactivational properties", BIOCHIMICA ET BIOPHYSICA ACTA . GENE STRUCTURE AND EXPRESSION, ELSEVIER, AMSTERDAM, NL, vol. 1759, no. 6, 1 June 2006 (2006-06-01) , pages 308-318, XP025034435, ISSN: 0167-4781, DOI: 10.1016/J.BBAEXP.2006.04.009 [retrieved on 2006-06-01]
- CHEN ZHILEI ET AL: "Directed evolution of human estrogen receptor variants with significantly enhanced androgen specificity and affinity", JOURNAL OF BIOLOGICAL CHEMISTRY, vol. 279, no. 32, 6 August 2004 (2004-08-06), pages 33855-33864, ISSN: 0021-9258

## Description

The present invention relates to a method for manufacturing a modified polypeptide from a first polypeptide, said modified polypeptide exhibiting altered binding properties to a target molecule and/or having a different amino acid sequence compared to a first polypeptide.

Most biological processes involve permanent and non-permanent interactions between different proteins. The development of modulators of protein-protein interactions possesses significant potential for the discovery of novel protein-affinity reagents, which can be used for a variety of diagnostic and therapeutic purposes as well as for purification, imaging and reagent purposes. Reagents that disrupt protein-protein interactions must contend with a protein surface that is comparatively large, poorly defined and solvent exposed, and the scope of their targets shall not be limited by the immune response.

The challenges faced in developing molecules interfering with protein-protein interactions are the lack of small molecule starting points, the apparent nondescript nature of the target area on the surface of the molecule, the difficulty of distinguishing artefactual binding from real associations and the insufficiency of chemical libraries to obtain high-affinity binders by screening.

In US 2003/186385 methods for the identification of polypeptides able to bind to a target protein are disclosed. These methods involve the use of cells which are capable to express to chimeric polypeptides one of which being the target protein for which a binding protein shall be identified.

It is an object of the present invention to provide a method in which binding partners of a target molecule are identified and in which binding properties of the binding partners are modified in a process which is performed in an environment close to nature.

Thus, the present invention relates to a method for manufacturing a modified polypeptide from a first polypeptide, said modified polypeptide exhibiting an altered binding affinity to a target molecule compared to a first polypeptide, comprising the steps of:
a) providing a first cell comprising a nucleic acid molecule encoding for a first fusion polypeptide, said first fusion polypeptide comprising at least one first polypeptide and a transcriptional activation domain, and comprising a nucleic acid molecule encoding for a second fusion polypeptide, said second fusion polypeptide comprising the target molecule or a polypeptide domain binding the target molecule and a DNA binding domain, whereby the cell further comprises a reporter gene encoding a reporter polypeptide operably linked to an upstream transcriptional regulatory sequence comprising a DNA binding site for the DNA binding domain of the second fusion polypeptide,
b) cultivating the cells of step a),
c) identifying and pooling cells expressing the reporter polypeptide,
d) isolating the nucleic acid molecules encoding for the at least one first polypeptide of the pooled cells of step c),
e) modifying the nucleic acid molecules of step d) by introducing at least one mutation thus obtaining modified nucleic acid molecules encoding for a library of modified polypeptides,
f) introducing the modified nucleic acid molecules of step e) into at least one second cell comprising a nucleic acid molecule encoding for a second fusion polypeptide, said second fusion polypeptide comprising the target molecule or a polypeptide domain binding the target molecule and a DNA binding domain, and
g) repeating steps a) to f) at least two additional times until a nucleic acid molecule encoding for a modified polypeptide is obtained and isolated in step d) exhibiting at least 20% higher binding affinity to the target molecule compared to the first polypeptide, wherein in the repeating steps a) to d) the first polypeptide is exchanged with the library of modified polypeptides of step e).

The present method adapts the method of yeast 2 hybrid (Y2H) to the field of the in vitro evolution. It was rather surprising that the Y2H system is usable at all in such a system due to the low transformation rate of eukaryotic cells, such as yeast, which is less than a tenth or a hundredth of usual in vitro evolution systems using e.g. bacteria or phages. Moreover, Y2H was not regarded as a sophisticated system which is normally applied in in vitro evolutions. Quite contrary to usual in vitro evolution methods, Y2H is a rather basic method.

The method of the present invention allows to identify and to manufacture modified polypeptides derived from a first polypeptide, which show altered binding properties to a target molecule (e.g. protein, polypeptide, peptide, nucleic acid, carbohydrate) compared to the first polypeptide or which show comparable or substantially identical binding properties but have a different amino acid sequence. The latter features of a modified polypeptide are of particular interest when, e.g. in the course of an immune therapy, a polypeptide is required which shows a binding affinity and/or specificity which is similar to a naturally occurring polypeptide and should be recognized as "foreign" by the immune system of a mammal.

A main feature of the method of the present invention is the use of iterative steps leading from a first polypeptide to a modified polypeptide exhibiting different properties compared to the first polypeptide. In the course of said method steps a) to f) is repeated at least two additional times (so that steps a) to f) are performed at least three times), preferably at least three, four, five, ten, 20, 30 etc. times. In the course of the method (after step f) the at least one first polypeptide of step a) is the modified polypeptide obtained in step e). Consequently, due to the use of repeating steps a polypeptide is subjected to an in vivo/in vitro evolution, wherein the binding properties of the first/modified polypeptide are determined in vivo and the mutagenesis ("evolution") is performed in vitro. Of course, the terms "in vivo" or "in vivo selection" in the present method refer to the steps performed in living cells, such as single cell organisms, like yeast or bacteria, or in cells or cell cultures or tissue of complex organisms, such as human or animal cells or cell lines. The "synthetic in vivo mutagenesis" according to the present invention is therefore also possible and covered in cell culture of e.g. human cells. According to the present invention, these repeating steps wherein the polypeptide is subjected to mutagenesis and selection procedures, wherein the binding properties of the modified polypeptide are determined, are performed in a living cell (in an intracellular environment).

The method of the present invention allows to select iteratively and subsequently to produce affinity binders in an intracellular environment. It is particularly advantageous that the manufacturing of a modified polypeptide showing an altered binding property to a target molecule occurs intracellularly. Every protein-protein interaction requires extracellularly certain interaction conditions like pH, salt concentration, temperature, working and binding conditions, detergents etc. which have to be maintained in the course of the determination of the protein-protein interaction and which do not reflect the naturally occurring conditions. The maintenance of these defined conditions in vitro is laborious and requires high accuracy. With the method of the present invention and with the provision of an in vivo method to manufacture modified polypeptides having altered binding properties to a target molecule compared to an unmodified polypeptide these drawbacks can be overcome. With the method of the present invention the binding conditions are reproducibly predetermined by the cell itself. Another main advantage of the present invention is that the polypeptide-target interaction occurs in a reducing intracellular environment. In contrast thereto, conventional in vitro methods like phage, ribosome, bacterial and yeast display technologies occur in an oxidising environment (in vitro). Since many therapeutic substances act intracellularly when administered to an individual or animal, it is advantageous that these substances which may be the modified polypeptides of the present invention are identified and obtained by a method in an intracellular and thus reducing environment. The binding properties of polypeptides to a target molecule may vary in an oxidising and reducing environment. The end result of the selection is a bank of binding peptides, rather than single molecular entities. Each target and binder combination is trapped within a single cell.

The method of the present invention overcomes various drawbacks of biochemical methods known in the art which are regularly used to manufacture modified polypeptides exhibiting an altered binding affinity to a target molecule compared to the unmodified versions of said polypeptides. Usually the target molecule as well as the polypeptides to be tested and manufactured have to be recombinantly produced and purified. The recombinant production and in particular the isolation of polypeptides is usually very laborious (comprising the steps of cloning of the polypeptides, expression and isolation of the polypeptides and testing the binding behaviour of the polypeptides to the target molecule) and expensive and therefore not suited for a fast and routinely used identification method of modified polypeptides showing altered binding properties to a target molecule compared to unmodified polypeptides. By using the method of the present invention it is possible to determine in vivo whether a modified polypeptide exhibits altered binding properties to a target molecule compared to an unmodified polypeptide. Therefore, there is no need to isolate the modified polypeptides from the cells in order to determine the binding properties of the modified polypeptides to a target molecule. This allows also screening a high number of modified polypeptides.

The method of the present invention allows to detect in vivo whether a modified polypeptide binds to the target molecule or not and - if the expression rate of the reporter polypeptide is quantified - to determine the binding strength.

In contrast thereto in vitro systems require the isolation of a modified polypeptide prior to determining its binding properties to a target molecule. Thus, the detection of the expression of the reporter polypeptide in vivo according to the present invention ("directed in vivo mutagenesis" or "synthetic in vivo mutagenesis") measures the result of the selection process during the mutagenesis and selection procedure, and can be used by a skilled artisan to determine whether further mutagenesis and selection steps need to be performed.

A further advantage of the present method is the fact that the modified polypeptide is expressed and bound to the target molecule in a natural defined and constant environment so that the binding conditions are highly reproducible, so that there is no need to optimize additionally the binding conditions in an in vitro test assay.

The modified polypeptides manufactured with the present method preferably exhibit a stronger binding (higher affinity) and/or higher specificity to a target molecule compared to the unmodified polypeptides, whereby these properties may preferably be determined by determining the reporter activity (e.g. LacZ, HIS3, ADE2, URA3, GusA etc.; such methods are well known in the art e.g. Serebriiskii IG et al. Biotechniques. 2000 29:278-9, 282-4, 286-8 and Estojak J. et al. (1995) Mol. Cell.Biol. 15:5820-5829). Of course, it is also possible to determine the reporter activity outside the cells (in vitro) by appropriate biochemical and genetic methods. This may be achieved by lysing the cells and by determining the activity of the reporter polypeptide in e.g. microtiter plates or by an e.g. ELISA assay. Alternatively, the reporter activity may preferably be determined by quantitative methods involving e.g. β-galactosidase (LacZ) or qualitative methods involving antibiotic resistance (e.g. HIS3) or antibiotic sensitivity (e.g. URA3). The latter method may preferably be used to manufacture polypeptides which show a reduced binding to the target molecule.

The binding properties may be optimized by performing several cycles comprising method steps a) to f) until at least one modified polypeptide is identified showing predetermined properties (e.g. altered binding properties to the target molecule compared to the at least one first polypeptide and/or having a different amino acid sequence compared to the first polypeptide). In practice the iterative steps of the method of the present invention will be preferably applied until the activity of the reporter polypeptide reaches a certain level. For instance, the steps may be repeated until the reporter activity recorded on a chart in each step reaches a plateau.

According to the present invention the iterative steps of the method will be repeated until the modified polypeptide(s) obtained exhibit "predetermined" properties (e.g. binding affinity). This means that the iterative steps of the method will be repeated until the modified polypeptide(s) obtained exhibit "preferred (or: desired)" properties (e.g. an optimised binding affinity or an alteration of solubility with constant binding affinity). This can be done by observing the evalution of the polypeptide during the repeats and repeating until no variation is visible anymore (this, of course, depends on the detection limits of the measurement means) or until an initially predetermined value has been achieved. On the other hand, the predetermined property can also be a qualitative property relative to the initial polypeptide (e.g. at least 20% higher (or lower) binding affinity or at least 20% lower (or higher) molecular weight (or pl-value, hydrophilicity, etc.) under preservation of binding affinity).

These properties may vary, among others, from polypeptide to polypeptide and depend on the use of the modified polypeptides obtained by the method of the present invention. The desired properties may be predetermined by the skilled artisan prior the application of the present method.

In the prior art, some reports disclose that Y2H has been used to change polypeptides, however, Y2H has never been suggested as a tool for performing in vitro evolution: Williams et al. (NAR 33 (2005), 4475-4484) performed a classic Y2H screen (according to Fields and Song) with HOXA13 and identified Smad5 as interacting protein from a limb bud cDNA library. A fragment encoding amino acids 175-465 from Smad5 was isolated. This Smad5 coding sequence was reintroduced in a Y2H bait vector and retested for confirmation of the interaction with a fragment of HOXA13 (amino acids 150-360), HOXD13 (amino acids 1-312), HOXA11 (amino acids 1-281) and HOXA9 (amino acids 1-245). Then, to identify the Smad5 domains that interact with HOXA13, deletion constructs of Smad5 were tested for interaction. Smad5 clones (amino acids 1-198, 146-198, 1-265) failed to interact with HOXA13, whereas Smad5 clones (amino acids 146-465, 202-465, 265-465) could interact.

In summary, Williams et al., a) performed a classic Y2H screen with the well known lexA-Y2H system in a cDNA library, b) identified Smad5 as interaction partner, c) re-introduced the interactor into the Y2H system to confirm the interaction, d) used specific deletion mutants of Smad5 to map the HOXA13 interacting domain.

In contrast thereto, the present invention provides a new interaction and clarified the part of the molecule that is important for interaction. In the prior art, Y2H was used for analysis but not for engineering new molecules with altered molecular properties. For example, there was no intention at all of Williams et al. to develop new molecules that have higher affinity to a HOXA13.

According to the present invention repeated use of mutagenesis/selection/monitoring of interaction strength, which is called "in vitro evolution" (*de novo in vitro* engineering of strong binders), enables engineering of new molecules by using the classic Y2H system. After the combination of several rounds of mutagenesis and selection and monitoring, the polypeptides thus generated are changed in their amino acid composition and have increased affinity to a target. The generated molecules are not naturally occurring ones. Williams et al., did neither use repeated mutagenesis & selection nor did they investigate and analyse nor produce novel molecules with increased affinities and amino-acid composition. Performing the "*in vitro evolution*" according to the present invention with the Y2H system may encompass the screening with Y2H and specific bait, mutagenesis, retesting with the Y2H system, measurement of binding affinity (= monitoring of protein strength indicators/reporters), iteration of these steps until no further change in the molecular property is detectable. The end result of this method is an increased affinity of the molecule compared to the original molecule and identification and production of a new peptidic molecule, not naturally occurring, and a strong binder of the target molecule.

Several authors observed a Y2H interaction between Bem1 and Cdc42 in former studies. For example Yamaguchi et al. (JBC 282 (2007): 29-38) examined various portions of Bem1 to identify the part that is responsible for Cdc42 binding. To identify the interacting domain of Bem1, the authors cloned a series of Bem1 fragments into a Y2H prey vector. The Bem1 fragments (amino acid 1-551, 1-140, 1-256, 140-551, 283-551, 140-256) were tested for interaction in the Y2H system. The Bem1 fragment 140-256, which contains a SH3 domain could specifically interact with Cdc42. Since this portion of Bem1 also interacts with Ste20, the authors decided to pinpoint the region to identify amino acid residues responsible for Cdc42 binding. By using a dual-bait Y2H system (a special type of the Y2H system that enables simultaneous screening of 2 different baits, here Ste20 and Cdc42 for Bem1 fragment 140-246) they searched for mutants, which fail to interact with Cdc42 but not with Ste20. Yamaguchi et al. performed an error-prone PCR and subsequently a dual-bait Y2H screening. Using this strategy they isolated 3 different mutants that interact with Ste20 but fail to interact with Cdc20.

Yamaguchi et al. therefore used the information of a published classic Y2H analysis, the protein interaction between Bem1 and Cdc42 for a detailed study; revealed a small portion of Bem1 to interact with Cdc42 by testing 6 deletion mutants of Bem1 in a classic Y2H system and mutagenized this fragment by error-prone PCR and screened for loss of function mutants (unable to bind to Cdc42) in a dual-bait Y2H system and identified three mutants that failed to interact to Cdc42.

Accordingly, Yamaguchi et al. used an already known Y2H interaction and clearly identified the part of the molecule that is important for interaction. Then they mutagenized and identified amino acid residues essential for binding to Cdc42. There was no intention at all to produce new molecules with higher affinity to Cdc42. I.e. they analysed and did not engineer new molecules with enhanced molecular properties with respect to binding, they did not try to enhance the performance or affinity of molecules.

In the method according to the present invention, after several rounds of mutagenesis and selection and monitoring (at least three rounds), the polypeptides thus generated are changed in their amino acid composition and increased in affinity to a target. The repetition of mutagenesis and selection generates new molecules, and a "gain of function" (here: the property to bind stronger to the target) enables the cells to survive (here: the cell harbouring the strong binder can grow under strong selection pressure) and therefore can enter a new round of mutagenesis and selection. "Loss of function" mutants are eliminated because they are outperformed during "in vitro evolution". The molecules generated with the procedure according to the present invention are not naturally-occurring ones, their amino acid composition is changed and binding strength is increased relative to their "ancestors" existing in nature.

Yamaguchi et al. did neither continuously repeat mutagenesis and selection and monitoring nor produced novel molecules with increased affinities; of course, as evidenced above, they did not perform "in vitro evolution" with the Y2H system.

In Williams et al., Yamaguchi et al. and others (e.g. Drees et al. JBC 154 (2001): 549-571; Allen et al. TIBS 20 (1995): 511-516; Pajunen et al. NAR 35 (2007): 3-5; WO 00/66722; EP 1 405 911 A1 or WO 02/31165 A1), an "extended classic Y2H analysis" is used which comprises: Taking a bait protein, look for interactors in a gene library and identify an interacting protein; then the Y2H analysis is extended by mutagenesis of either the bait or the prey molecule (one of the interacting proteins) to map the domains responsible for binding. These results in identification of interacting portions (domains) of the interactor, a search for mutants unable to interact, together aiming at a molecular analysis of the interacting process.

In contrast thereto, the present invention adds an in vitro evolution step to the classic Y2H analysis and the extended Y2H analysis which includes the monitoring of interaction strength after mutagenesis and selection, repetition of the steps as long as an increase in binding is detectable to obtain molecules with increased affinities or desired properties.

Thus, whereas classic Y2H analysis delivers a pair of candidate interacting proteins and extended Y2H analysis delivers a protein-fragment or domain responsible for binding to a desired target, the in vitro evolution according to the present invention yields novel molecules with increased affinity, an engineered property, to a desired target molecule, as a result of repeated rounds of mutagenesis + selection + monitoring.

Further advantages of the method of the present invention are:
● Sequence information is sufficient to archive and recreate
● Mutagenesis and selection can be applied
● Functional domains can be fused to tags for downstream applications
● End products can be made by chemical synthesis or with bacterial expression systems
● Selection yields many binders per targets or many targets per binders
● Many targets are tested against many binders in one experiment (rather than one target and many binders)
● One binder and one target occur per cell, which can be identified by sequencing

The nucleic acid molecule encoding for a first fusion polypeptide and optionally the nucleic acid molecule encoding for a second fusion polypeptide are introduced in the first cell provided in step a) by using preferably a vector (e.g. bacterial, yeast or viral vector) through transfection or transformation or by directly transforming the cell with the linear nucleic acid molecules. In both cases and in particular in the latter case the nucleic acid molecule may be provided with a region which allows a (preferably stable) homologous recombination of said nucleic acid molecule into the genome of the cell.

If the modified polypeptide of the present invention is or should be capable to bind to a specific binding site on a nucleic acid molecule it is sufficient that said first cell comprises only a reporter gene encoding a reporter polypeptide operably linked to an upstream transcriptional regulatory sequence to which the first and modified polypeptide are capable to or should bind. If such a binding event occurs the transcriptional activation domain of the first fusion polypeptide induces the transcription and consequently the biosynthesis of the reporter polypeptide. Of course the upstream transcriptional regulatory sequence should comprise the nucleotide sequence to which the first and/or modified polypeptide is capable to bind to. Nevertheless it is of course also possible to provide a cell comprising next to a nucleic acid molecule encoding for a first fusion polypeptide a nucleic acid molecule encoding for a second fusion polypeptide as defined above. In this case, however, both the first and the second fusion polypeptide may bind to the same nucleic acid molecule on which the binding site for the first fusion polypeptide can be found.

The reporter gene encoding a reporter polypeptide operably linked to an upstream transcriptional regulatory sequence may be present in said first cell on an extrachromosomal vector or integrated on the chromosome.

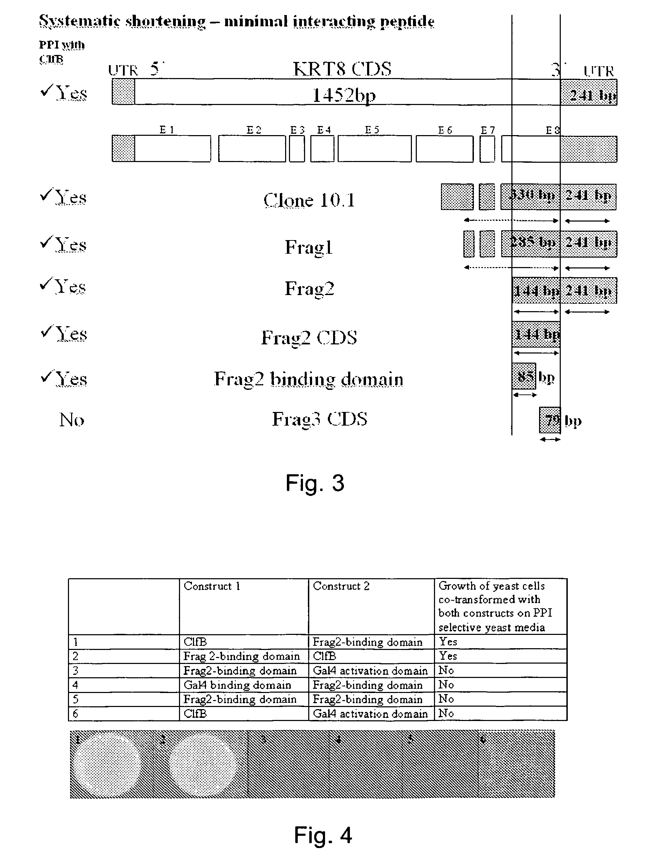

The first cell may, however, also comprise a nucleic acid molecule encoding for a second fusion polypeptide, which comprises the target molecule (e.g. enzyme, receptor) or a polypeptide domain binding to the target molecule (e.g. carbohydrate structure, nucleic acid molecule, polypeptide, organic compound) and a DNA binding domain, which is capable to bind to the upstream transcriptional regulatory sequence of the reporter gene. The presence of said second fusion polypeptide within the first cell is particularly desired when the first or the modified polypeptide is or should be capable to bind directly to the target molecule or when other molecules (e.g. chemical compounds, carbohydrates, polypeptides, nucleic acid molecules) are the target molecule and the second fusion polypeptide comprises a domain capable to bind also to the target molecule directly or via further molecules. Similar concepts are known from the yeast hybrid system (e.g. one-, two and three hybrid system, see e.g. Hollingsworth R et al. (2004) DDT:Targets 3:97-103, in particular Fig. 4).

Using the terminology of the yeast two hybrid system (see e.g. Hollingsworth R et al. (2004) DDT:Targets 3:97-103) the first fusion polypeptide may be denominated as prey fusion protein/polypeptide and the second fusion polypeptide as bait fusion protein/polypeptide. Consequently, first fusion polypeptide may be used interchangeable with prey fusion protein/polypeptide and second fusion polypeptide with bait fusion protein/polypeptide.

The bait fusion protein includes a fusion between a polypeptide moiety of interest (e.g., a protein of interest or a polypeptide from a polypeptide library), and a DNA-binding domain which specifically binds a DNA binding site which occurs upstream of an appropriate reporter gene. The nucleotide sequence which encodes the polypeptide moiety of interest is cloned in-frame to a nucleotide sequence encoding the DNA-binding domain.

Any polypeptide that binds a defined DNA sequence can be used as a DNA-binding domain. The DNA-binding domain can be derived from a naturally occurring DNA-binding protein, e.g., a prokaryotic or eukaryotic DNA-binding protein. Alternatively, the DNA-binding domain can be a polypeptide derived from a protein artificially engineered to interact with specific DNA sequences. Examples of DNA-binding domains from naturally occurring eukaryotic DNA-binding proteins include p53, Jun, Fos, GCN4 or GAL4. The DNA-binding domain of the bait fusion protein can also be generated from viral proteins, such as the pappillomavirus E2 protein. In another example, the DNA-binding domain is derived from a prokaryote, e.g. the E. coli LexA repressor can be used, or the DNA-binding domain can be from a bacteriophage, e.g., a lambda cl protein. Exemplary prokaryotic DNA-binding domains include DNA-binding portions of the P22 Arc repressor, MetJ, CENP-B, Rap1, Xy1S/Ada/AraC, Bir5 and DtxR.

The DNA-binding protein also can be a non-naturally occurring DNA-binding domain and can be generated by combinatorial mutagenic techniques. Methods for generating novel DNA-binding proteins which can selectively bind to a specific DNA sequence are known in the art (e.g. US 5,198,346).

The basic requirements of the bait fusion protein include the ability to specifically bind a defined nucleotide sequence (i.e. a DNA binding site) upstream of the appropriate reporter gene. The bait fusion protein should cause little or no transcriptional activation of the reporter gene in the absence of an interacting prey fusion protein. It is also desirable that the bait not interfere with the ability of the DNA-binding domain to bind to its DNA binding site.

As appropriate, the DNA-binding domain used in the bait fusion protein can include oligomerization motifs. It is known in the art that certain transcriptional regulators dimerize. Dimerization promotes cooperative binding of the transcriptional regulators to their cognate DNA binding sites. For example, where the bait protein includes a LexA DNA-binding domain, it can further include a LexA dimerization domain; this optional domain facilitates efficient LexA dimer formation. Because LexA binds its DNA binding site as a dimer, inclusion of this domain in the bait protein also optimizes the efficiency of binding. Other exemplary motifs include the tetramerization domain of p53 and the tetramerization domain of BCR-ABL.

The nucleotide sequences encoding for the bait and prey fusion proteins are inserted into a vector such that the desired bait fusion protein can be produced in a host cell. Suitable recombinant expression vectors are known in the art. Preferably the recombinant expression vectors may include one or more regulatory sequences operably linked to the fusion nucleic acid sequence to be expressed. The term "regulatory sequence" includes promoters, enhancers and other expression control elements (e.g., polyadenylation signals) etc.. Optionally, the vector can also include a selectable marker, the expression of which in the host cell permits selection of cells containing the marker gene from cells that do not contain the marker gene. Selectable markers are known in the art, e.g. neomycin, zeocin or blasticidin.

The vectors encoding for the bait and prey fusion proteins are preferably integrated into a chromosome of a cell.

It may also be preferred to introduce an unstructured polypeptide linker region between the DNA-binding domain of the fusion protein and the bait polypeptide sequence. The linker can facilitate, e.g., enhanced flexibility of the fusion protein allowing the DNA-binding domain to freely interact with the DNA binding site.

The prey fusion protein includes a transcriptional activation domain and a candidate interactor polypeptide sequence which is to be tested for its ability to form an intermolecular association with the bait polypeptide. As discussed above, protein-protein contact between the bait and prey fusion proteins (via the interaction of the bait and prey polypeptide portions of these proteins) links the DNA-binding domain of the bait fusion protein with the activation domain of the prey fusion protein, generating a protein complex capable of directly activating expression of the reporter gene.

Any of a number of activation domains can be used in the prey fusion protein. The activation domain can be a naturally occurring activation domain, e.g., an activation domain that is derived from a eukaryotic or prokaryotic source. Exemplary activation domains include GAL4, VP16, CR2, B112, or B117. The activation domain can also be derived from a virus, e.g., VP16 activation domain is derived from herpesvirus.

DNA sequences which encode the prey and the transcriptional activation domain, e.g., a VP16 activation domain, can also include other sequences such as a nuclear localization sequence (e.g., those derived from GAL4 or MATα2 genes). The nuclear localization sequence optimizes the efficiency with which prey proteins reach the nuclear-localized reporter gene construct.

The prey polypeptide can be any polypeptide, e.g., the prey polypeptide can be derived from all or a portion of a known protein or a mutant thereof, all or a portion of an unknown protein (e.g., encoded by a gene cloned from a cDNA library or an ORFeome), or a random polypeptide sequence.

To isolate DNA sequences encoding novel interacting proteins, members of a DNA expression library (e.g., a cDNA or synthetic DNA library) can be fused in-frame to the transcriptional activation domain to generate a variegated library of prey fusion proteins.

In an exemplary embodiment, a cDNA library may be constructed from an mRNA population and inserted into an expression vector. It is also noted that prey polypeptides need not be naturally occurring full-length proteins. In certain embodiments, prey proteins can be encoded by synthetic DNA sequences.

DNA sequences which encode for the prey protein and the activation domain, e.g., the nucleic acid sequence which encodes for the VP- 16 activation domain, are inserted into a vector such that the desired prey fusion protein is produced in a host mammalian cell. The vector can be any expression vector as described above. In the instance where it is preferable to recover the prey sequence using a bacterial host cell, as described above, the prey DNA sequences are inserted into a vector which contains an appropriate origin of replication. By an appropriate origin of replication an origin of replication is meant which allows the vector to be maintained episomally and indefinitely without damaging the mammalian host cell or integrating the DNA sequence into the genomic DNA of the mammalian host cell. Since the vector is maintained episomally, the vector can be easily introduced and recovered from a bacterial host cell. An example of such a suitable origin of replication is the oriP Epstein Barr virus replication origin sequence (oriP). In a preferred embodiment, a vector containing an oriP is transformed into a mammalian cell which contains an Epstein Barr virus nuclear antigen-1 (EBNA-1). A vector containing an oriP can replicate stably in a mammalian cell that expresses EBNA-1 (Aiyar et al., EMBO Journal, 17:12:6394-6403).

Expression of the reporter gene indicates an interaction between the prey and bait polypeptides, and permits the identification of mammalian cells in which an interaction has occurred. The reporter gene sequence will include a reporter gene operably linked to a DNA binding site to which the DNA-binding domain of the bait fusion protein binds.

In a preferred embodiment of the invention, the reporter gene encodes a fluorescent molecule, e.g., a green fluorescent protein (GFP) or a blue fluorescent protein (BFP). The advantage of using a reporter gene that encodes a fluorescent protein is that a single individual fluorescent positive cell can be identified quickly. For example, using GFP as the reporter gene product, green fluorescence can be detected as early as 16 hours after transfection. Positive (fluorescent) cells can be identified using a fluorescence microscope, e.g., using an inverted phase-contrast microscope equipped with an epifluorescence light source and a fluorescein isothiocyanate filter set. Using this method, positive cells can be identified without damage to the cells, e.g., positive, green fluorescent cells can be easily isolated by conventional cell cloning methods, such as using small plastic cylinders to isolate cells, or collecting positive cells directly using a conventional micropipette. Alternatively, fluorescence-activated cell sorter (FACS) can be used to isolate positive cells. However, isolating positive cells by FACS is less preferable, since this approach will mix up the positive clones, and hence, may cause cloning bias. The total DNA from a positive clone can be prepared by standard procedures and the sequence which encodes the prey protein amplified using PCR and sequenced by standard procedures. A preferred fluorescent polypeptide is derived from a GFP.

Of course, in the method of the present invention any suitable reporter gene can be used. Examples include chloramphenicol acetyl transferase (CAT; Alton and Vapnek (1979), Nature 282:864-869), and other enzyme detection systems, such as beta-galactosidase; firefly luciferase (deWet et al. (1987), Mol. Cell. Biol. 7:725-737); bacterial luciferase (Engebrecht and Silverman (1984), PNAS 1:4154-4158; Baldwin et al. (1984), Biochemistry 23:3663-3667); phycobiliproteins (especially phycoerythrin); alkaline phosphates (Toh et al. (1989) Eur. J Biochem. 182:231-238, Hall et al. (1983) J. Mol. Appl. Gen. 2:101), secreted alkaline phosphate (Cullen and Malim (1992) Methods in Enzymol. 216:362-368) or fluorescent proteins (e.g., GFP). Other examples of suitable reporter genes include those which encode proteins conferring drug/antibiotic resistance to the host cell.

The amount of transcription from the reporter gene may be measured using any suitable method. Various suitable methods are known in the art. For example, specific RNA expression may be detected using Northern blots, or specific protein product may be identified by a characteristic stain or an intrinsic activity.

In preferred embodiments, the protein encoded by the reporter is detected by an intrinsic activity associated with that protein. For instance, the reporter gene may encode a gene product that, by enzymatic activity, gives rise to a detection signal based on, fluorescence, colour, or luminescence.

The term "polypeptide", as used herein, refers to a proteinaceous molecule comprising at least 5 (preferably at least 7, more preferably at least 10 etc.) amino acid residues. The link between one amino acid residue and the next is an amide bond. The term "polypeptide" according to the present invention includes also peptides and proteins, terms used commonly in the art.

According to a preferred embodiment of the present invention the nucleic acid molecule encoding for the first fusion polypeptide and/or the nucleic acid molecule encoding for the second fusion polypeptide are comprised on at least one vector.

The nucleic acid molecules encoding for the first and the second fusion polypeptides may be present on a single vector, on more than one vector (preferably 2) or one or both nucleic acid molecules are integrated into the chromosomal/genomic DNA. Suitable vectors are knwon to the person skilled in the art and may be chosen depending on the host cell used and whether an integration into the genome of the host cell is desired.

In order to determine the properties of the first and the modified polypeptides the expression rate of the reporter polypeptide of the at least one cell identified in step c) is quantified. This quantification may occur directly (e.g. fluorescence) or indirectly (e.g. cell growth (cell density)). The quantification method depends on the reporter polypeptide used.

According to a preferred embodiment of the present invention the target polypeptide is selected from the group of receptors, structural proteins, transport proteins and enzymes, preferably cell surface receptors, transcription factors, and enzymes such as kinases, proteinases, phosphatases, other hydrolases, or translocases.

The bait portion of the bait fusion protein may be chosen from any protein of interest and includes proteins and/or polypeptides of unknown, known, or suspected diagnostic, therapeutic, or pharmacological importance. For example, the protein of interest can be a protein suspected of being an inhibitor or an activator of a cellular process (e.g. receptor signalling, apoptosis, cell proliferation, cell differentiation or import or export of toxins and nutrients). Examples of bait proteins include receptors, such as hormone receptors, neurotransmitter receptors, metabotropic receptors, ionotropic receptors and hormone receptors, oncoproteins such as myc, Ras, Src and Fos, tumor-suppressor proteins such as p53, p21, p16 and Rb (Knudsen et. al., Oncogene, 1999, 18:5239-45), proteins involved in cell-cycle regulation such as kinases and phosphates, or proteins involved in signal transduction, like T-cell signalling, e.g. Zap-70 or SAM-68. Usually the full length of the protein of interest is used as the bait protein. In cases where the protein of interest is of a large size, e.g. has a molecular weight of over 20 kDa, it may be more convenient to use a portion of the protein.

According to a preferred embodiment of the present invention the nucleotide sequences encoding the at least one first polypeptide are obtained from peptide and/or polypeptide libraries and/or from cDNA, genomic DNA or Expressed Sequence Tags (ESTs) of at least one organism and/or derived from the ORFeome of at least one organism and/or from artificial genomic or artificial nucleic acid libraries. In this context "organisms" include also viruses.

The prey moiety (i.e. at least one first polypeptide) of the prey fusion protein may be any polypeptide comprising at least 5 amino acid residues. The polypeptide can be derived from several sources whereby it is especially preferred to use polypeptide/peptide libraries or polypeptides/peptides from an ORFeome.

The polypeptide/peptide library-based method of the present invention starts with consensus-sequence peptides derived from binding partners in a defined protein-protein interaction starting preferably from naturally selected binders occurring in a complete ORFeome in the format of an ORFomer library (in which an ORFomer library is a library containing peptides and polypeptides derived from an ORFeome). The polypeptides are transformed through mutation into optimized binders of the target molecules in a selection and production procedure in an intracellular environment.

"Orfeome" or "Orfome" is the totality of open reading frames (ORF) in an organism or virus. ORFs code for polypeptides and proteins and can be determined by sequence analysis of the nucleotide sequences of the organism or virus. This analysis may be facilitated by computer programs such as Gen-Scan. However, a proteome that includes ORFeome may also be characterized empirically from experimental data generated by techniques such as mass spectroscopy. An advantage of doing selection in a two hybrid system starting from an ORFeome and using ORFomers is, all and any binary combinations of binders and targets are obtained in single cells. So far, peptide- or protein-affinity reagents were obtained without considering the complete range of binding proteins from a complete ORFeome, whether homologous(from the same source as the target protein) or heterologous (from a different source than the target protein), that is without taking into consideration naturally selected sources of affinity binders

A cDNA library according to the present invention refers to a complete, or nearly complete, set of all the mRNAs contained within a cell or organism. cDNA is usually obtained by employing reverse transcriptase which will produce a DNA copy of each mRNA strand. Referred to as cDNA these reverse transcribed mRNAs are collectively known as the library.

cDNA libraries may be prepared from total or enriched Poly(A)+ single stranded mRNA that is converted into a double-stranded DNA copy of the message using reverse transcriptase. The cDNA fragments can be inserted into an appropriate plasmid, phage or cosmid vector for maintenance and cloning. The population of recombinant vectors will represent the entire set of expressed genes in the cell from which the RNA was isolated. One of the main advantages of using a cDNA library is that the introns are spliced out and the mRNA sequence can be used as a template to create cDNA to collect the preferred genes. Differently than standard cDNA libraries, in full-length cDNA libraries most of the cDNA represent complete original mRNA molecules. These are produced by implementation of normal methods for cDNA libraries preparation. The main technologies are known as "cap-trapper" and "oligo-capping".

The organism from which the cDNA and the ORFeome are derived from is preferably a microrganism (including viruses), plant or animal, preferably a mammal.

According to a preferred embodiment of the present invention the at least one mutation of the nucleotide sequences of step e) is a point mutation, a deletion, an insertion, a DNA translocation, DNA shuffeling, DNA rearrangements and DNA multimerisation.

Due to the introduction of mutations into the nucleotide sequences encoding for the polypeptides or peptides binding to a target polypeptide (binders) and identified with the method according to the present invention, it is possible to change the properties of these binders in a way to create molecules exhibiting e.g. stronger binding affinity to the target polypeptide and thus having an increased inhibitory effect compared to the wild-type molecules. The introduction of mutations into the nucleotide sequences may be done with molecular biological methods known in the art.

It is in particular preferred to mutate the identified polypeptide by truncating (deleting amino acid residues from the N- and/or C-terminus) or fragmenting its amino acid sequence. This allows to identify e.g. small or even the smallest polypeptides still binding to the target polypeptides. Short polypeptides are often used as inhibitors of target polypeptides because they are able to bind to the targets without showing other biological effects or as being recognized as foreign by the immune system when administered to a mammal.

According to another preferred embodiment of the present invention the vector is a prokaryotic hybrid vector, preferably a bacterial hybrid vector, or a eukaryotic hybrid vector, preferably a yeast, insect or mammalian hybrid vector and/or the cell is a prokaryotic cell, preferably a bacterial cell, or a eukaryotic cell, preferably a yeast, insect or mammalian cell.

Depending on the two hybrid systems used, the vectors and the host cells are chosen appropriately.

The present invention is further illustrated by the following figures and examples, however, without being restricted thereto.
Fig. 1 shows that GST-tagged FnbB could be co-purified with a) HSPC 118, b) CCT - beta, c) lysosomal sialidase NEU 1, (d) beta - actin and (e) FLNA from a cell-free protein translation system (rabbit reticulocyte coupled transcription and translation system). Rectangles indicate the co-purified recombinant protein. Left lanes are pull-down experiments where GST-tagged recombinant FnbB was included. Right lanes are pull-down experiments where only recombinant GST-protein was included in the reactions as a negative control.

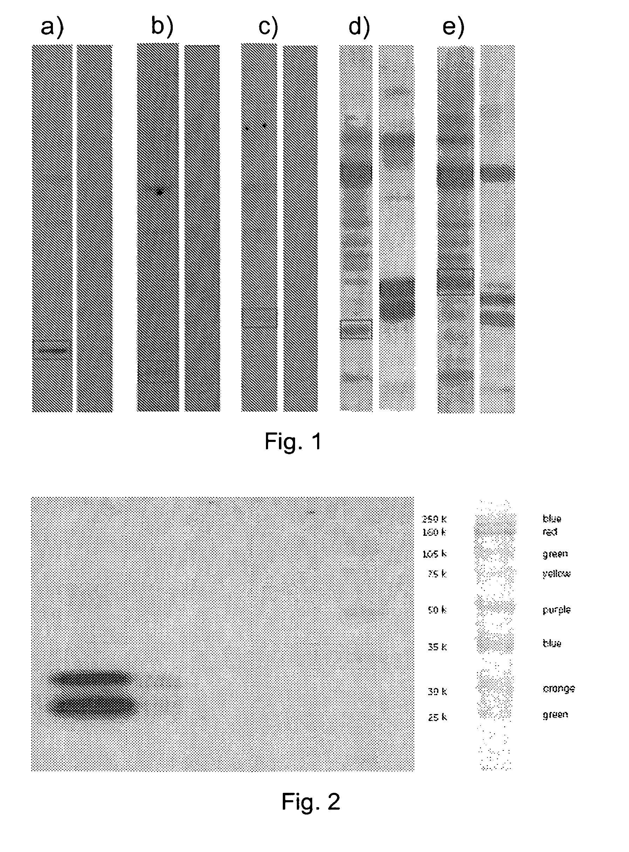

Fig. 2 shows that His-tagged recombinant CIfB protein could be co-purified with recombinantly expressed GST-tagged Keratin8 protein fragment (Frag2-CDS). Detection of the co-purified KRT8 protein fragment was done with anti-GST antibodies. Lane a) input of Keratin 8 protein fragment in each pull-down experiment, b) Keratin 8 protein fragment pulled-down with His-tagged CIfB protein immobilized on his-tag affinity magnetic beads, c) negative control: HIS-tagged CIfB protein was omitted from the pull-down analysis. D) RPN800 rainbow protein size marker. GST-tagged Keratin 8 fragment produced recombinantly in E. coli cells resulted in 2 differently sized proteins with approximately 33 kd and 30 kd. The recombinant GST-tagged Keratin 8 protein fragment could only be pulled down when His-tagged recombinant CIfB was present in the experiment
Fig. 3 shows the systematic shortening of Keratin 8 protein to a minimal interacting peptide.
Fig. 4 shows the results of binding as tested by the Y2H-system.
Fig. 5 shows that neither a) BSA nor c) GST coated beads can pull-down the GST-tagged peptide of 48-amino acids. Only His-tagged CIfB protein coated beads can precipitate the GST-tagged peptide of 48 amino acids.
Fig. 6 shows that recombinant CIfB could bind to the synthetic peptide IPEP21-SA as well as to the recombinant 48-amino acid-peptide (Frag2-CDS).
Fig. 7 shows a comparison of the smallest CIfB introducing fragment identified with the yeast two hybrid screen.

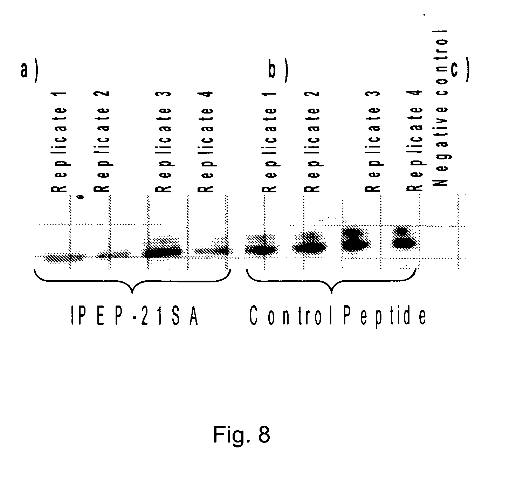

Fig. 8: a) in the presence of 1 µM specific peptide (IPEP-21 SA) the GST-tagged keratin fragment is pulled-down to a lesser extend as b) in the presence of control peptide. The negative control experiment was made without CIfB-protein (c). The concentration of 1 µM IPEP-21 SA inhibits competitively the binding of GST-tagged keratin fragment to CIfB-protein by ∼40%. Relative changes in pulled-down GST-tagged keratin fragment is quantified by densitometry using a flatbed scanner and the ImageJ software, provided by Wayne Rasband, National Institutes of Health. (ImageJ: httip://rsb.info.nih.gov/ij/)
Fig. 9: PPI-inhibition with escalating concentrations of specific and control peptides (> 100 µM). A) in the presence of IPEP-21 SA, b) in the presence of control peptide, c) negative control experiment without CIfB-protein. Arrow indicates keratin fragment pulled-down.

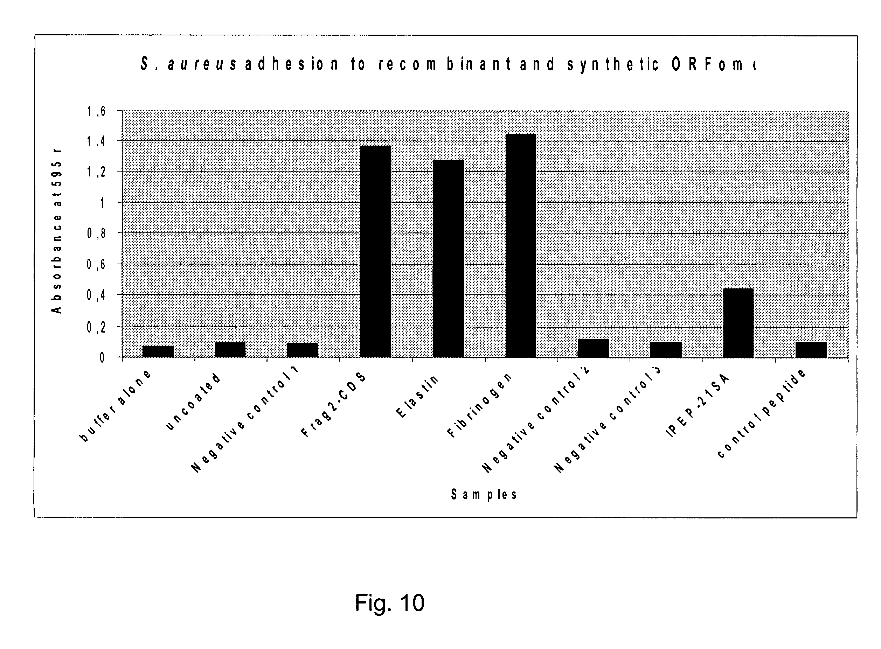

Fig. 10: recombinant GST-tagged keratin fragment (recombinant ORFomer) binds strongly to living pathogen cells with affinities comparable to published virulence factor substrates as shown by an adhesion assay. IPEP-21SA (synthetic ORFomer) also binds strongly to living pathogen cells compared to synthetic control peptide. 1 µg of the following proteins were coated onto microwell-plates and incubated with living ***S.aureus*** cells. Bound ***S. aureus*** cells were detected by crystall-violet staining and absorbance at 595 nm:
   a) Bovine serum albumine (BSA) (500ng/µl). This protein served as negative control 1; b) Affinity purified, recombinantly in ***E. coli*** produced GST-tagged Frag2-CDS. The amino acid sequence of Frag2-CDS is N-YSLGSSFGSGAGSSSFSRTSSSRAVVVKKIETRDGKLVSESSDVLPK-C; c) Human aortic elastin protein. This protein served as positive control 1 because elastin protein is known to bind to ***S. aureus;*** d) Human fibrinogen protein. This protein served as positive control 2 because fibrinogen protein is known to bind to ***S. aureus;*** e) Affinity purified, recombinantly in ***E. coli*** produced GST-tagged ***S. aureus*** protein, methionine sulfoxide reductase msrB, NCBI protein GI:54041496. This protein served as negative control 2; f) Affinity purified, recombinantly in ***E. coli*** produced GST-tagged ***S. aureus protein***, NADPH-dependent 7-cyano-7-deazaguanine reductase, NCBI protein GI:81781951. This protein served as negative control 3; g) Synthetic IPEP-21SA. The sequence of IPEP-21SA is is N-SYSLGSSFGSGAGSSSFSRTS; h) Synthetic control peptide, the sequence of the control peptide is N-EQRGELAIKDANAKLSELEAAL.

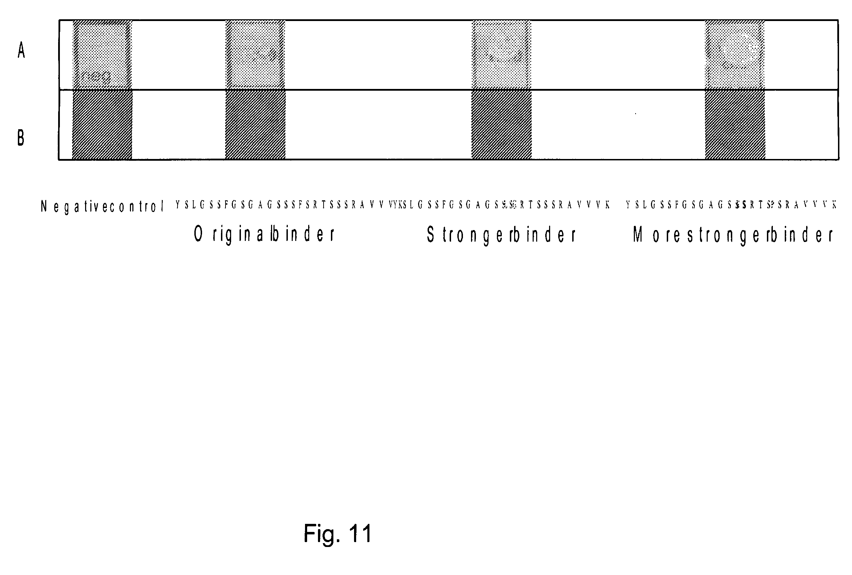

Fig. 11: engineered novel CIfB binding peptides by in vitro evolution in the Y2H system. The original binder is a portion of K8 which binds to CIfB. Stronger and more stronger binders are developped and resulted in non-natural peptides with higher affinity to CIfB after repeated rounds of mutgenesis/selection/monitoring.
   a) Comparison of growth strength on 50 mM 3-AT containing highly selective yeast media.
   b) Comparison of beta-galactosidase expression by X-Gal beta galactosidase overlay assays. Both measurements are an indicative for the relative protein-protein interaction strength in the Y2H system. Negative control shows no growth on 3-AT containing medium and no blue color development (no beta galactosidase expression). The stronger binders produce at least 50% and 100% more beta galactosidase in comparison to the original binder, respectively. Equal numbers of cells from each clone were incubated all plates. The growth of Y2H yeast cells (stronger binders harbouring clones) on 3-AT included selective medium increased significantly compared to the original binder.

### EXAMPLES:

The increasing incidence of antibiotic-resistant strains of *S.aureus* defines the need for alternatives to the current arsenal of antibiotics. To infect and colonize a host, and then cause disease, *S.aureus* uses several proteins, both own and host-derived. Protein-protein interactions (PPI) are the basis of this human - pathogen "conversation" resulting in infection and disease. Revealing the complete protein interface ('interactome') between *S. aureus* and its host (man) would offer targets for new therapeutic and diagnostic approaches. Hence a research objective has been the search for the human proteins interacting with *S. aureus* surface adhesins, which are bacterial proteins involved in early steps of the infection.

*S. aureus* can adhere to components of the extra cellular matrix (ECM) of the host. This is accomplished through surface expressed and cell wall anchored protein adhesins, so called 'microbial surface components recognising adhesive matrix molecules' (MSCRAMMs). A structural organization of MSCRAMMs is shown in Fig. 1. Adherence of *S. aureus* to.collagen, fibronectin, laminin, proteoglycans and elastin has been shown. Furthermore, Gram-positive bacteria such as S. aureus use surface molecules in subsequent pathogenic steps, such as evading host immune responses, digesting host carbohydrates to expose host attachment sites, capturing host enzymes to digest host tissues or binding host tissue factors to establish a firm basis for colonization. The following surface proteins are known to be involved in S.aureus pathogenesis, and function as adhesins:
*Protein A (Spa):* mediates adhesion to the van Willebrand factor, which is expressed at damaged sites of the host endothelium.
*Clumping factors (CIfA* + *B):* mediate adhesion and clumping of bacteria to fibrinogen.
*Collagen binding protein (Cna)*
*Elastin binding protein of S. aureus (EbpS)*
*Fibronectin binding proteins (FnbA and FnbB)*

Cna is able to bind to collagen substrates and collagenous tissues. Cna is not expressed by the majority of strains, and S.aureus strains which lack Cna expression are less virulent. A 55 kDa domain (AA 30 - 531) contains the collagen binding site in a 19 kDa sub domain (AA 151 - 318). Although the 19 kDa sub domain can bind to different types of collagen, (via a GPP containing triple helix in collagen), the 55 kDa domain shows a higher affinity to the substrate than the 19 kDa domain. A synthetic peptide mimicking this sub-domain inhibits collagen binding to bacteria.

EbpS is encoded by a 1461 bp ORF in the *S. aureus* strain MU50 (source: NCBI - database; gi: 47208328). It differs from the above MSCRAMMs by its cell wall anchor (no LPXTG motif in the cell wall anchor). EbpS can bind elastin, which is one of the major protein components of the ECM. Hot spots of elastin expression are the lung, the skin and the blood vessels. EbpS binds to the N -terminal 1/3 of elastin through its N - Terminal region (encoded by the first 609 bp). Recombinant EbpS, as well as a Fab fragment of an Ab which was raised against recombinant EbpS, inhibit binding of *S. aureus* to elastin.

FnbA and FnbB are expressed through two closely linked genes. FnbA and FnbB can bind immobilized fibronectin in vitro, and they contribute to the adherence of *S. aureus* to plasma clots and to surgical material that has been in contact with the host. The ligand binding domains (D domains) are located very close to the cell wall-spanning anchor in the C - terminal region of the surface protein. They consist of 3 - 5 repeats of a 37 - 38 AA motif. Individually, the domains can bind fibronectin with a low micromolar dissociation constant (K_{d}), in tandem they compose a high affinity domain with a dissociation constant of K_{d} = 1,5 nM. In addition to the D-domains, two other fibronectin-interacting domains have been identified: DuA and DuB. Peptides which mimic domains of the D - region inhibit fibronectin binding by the pathogen. The binding domain interacts simultaneously with multiple sites of the N - terminus of fibronectin.

The bacterial binding site of fibronectin is located in the N - terminus and consists of 5 sequential fibronectin Type 1 modules (F1: a beta - sandwich of 2 anti parallel beta - sheets). The main binding site for the binding D - domains of *S. aureus* is a F1F1 module pair. This domain is bound by ligand induced formation of additional anti parallel beta - strands in the binding domain of the FnbA/FnbB, which results in a tandem beta - zipper interaction. Electrostatic interactions are important and an anti parallel orientation for the beta - sheets of fibronectin

The aim of the following examples is to show that a protein interacting with another protein can be reduced to a "minimal interacting peptide" (i.e. the smallest peptide still binding specifically to the target protein), which can compete with the full length protein in binding to the target protein and therefore act as an inhibitor, as follows:
○ Using the Y2H system, identify human proteins, which interact with *S. aureus'* surface proteins
○ Using mutagenesis and selection in the Y2H system, derive peptides from the human proteins that bind to the bacterial proteins
○ Demonstrate that the peptides are specific binders of the Protein-Protein Interaction (PPI) addressed, including demonstration that the peptides inhibit the PPI addressed (i.e. are "PPI-inhibitory peptides")

### Experimental approach:

○ Construction of an ORFeome, exemplified by the ORFeome of ***S. aureus.*** An ORFeome is the requirement for construction of ORFomer libraries; see below
○ The staphylococcal surface proteins CIfB, Cna, FnbB and EbpS are cloned into Yeast 2 hybrid (Y2H) compatible vectors ("bait" libraries)
○ The Y2H system is used for testing of S. aureus bait libraries vs. a Y2H-compatible cDNA prey-library derived from human keratinocytes. The keratinocyte prey-library is produced from cultivated primary keratinocytes. The Y2H system is used to identify interacting proteins.
○ Protein-protein interactions are verified with technologies different from the Y2H system, namely immuno-precipitation (IP) and pull-down assays (PD).
○ Production of a peptide library from an ORFeome called unspecific (unspecific for binding to one or more targets) and unselected ORFomer library (unselected means that this library contains binders as well as non-binders)
○ Production of a specific peptide library from interacting proteins called specific (all plasmids of this library encode for a peptide, which was derived from one or more proteins, which can interact with one or more targets in the initial Y2H-screen) but unselected ORFomer library
   Exemplified by
○ Systematic mutagenesis: Shortening of the gene encoding the interacting protein, resulting in N- and C-terminal truncations, until the interaction is lost to identify the smallest peptide derived from the binding proteins still interacting with the target protein
○ Mutagenesis followed by selection and further rounds of mutagenesis & selection of minimal interacting peptides in an ORFomer library → selected or mature ORFomer specific for one target.
   Exemplified by
○ Random mutagenesis: A Y2H-compatible 'restriction-fragment gene-library' was produced from cDNA encoding human proteins interacting with a target protein, with the aim to identify (using the Y2H system) the smallest peptide derived from the binding proteins still interacting with the target protein. Chemical synthesis is used to confirm the nature of the binding peptide. The peptide can be used for a variety of applications.
○ Binding specificity of the peptides is shown by testing the binding capability to other proteins and peptides as well as binding of the peptide to itself. Proteins unlikely to be involved in target protein (in our case virulence factor) binding or unlikely to be a target of the peptide are tested in the Y2H-system. Additionally, self-aggregation capacity of the minimal-peptide is excluded by testing the ability to homodimerize in the Y2H-system. To exclude yeast system-based artefacts, the binding specificity with recombinant proteins by in vitro pull-down analysis was demonstrated.
○ Tested by Y2H-technology:
   The binding of the peptide to a yeast protein, the binding-domain of GAL4 protein (to exclude unspecific binding)
   The binding of the peptide to CIfB (specific binding)
   The binding of the peptide to the same peptide (stickiness, to exclude unspecific binding)
   The binding of CIfB to the peptide (specific binding)
   The binding of CIfB to a yeast protein, the activation-domain of GAL4 protein (to exclude unspecific binding)
○ Tested by in vitro pull-down analysis:
   The binding of the peptide to beads coated with the specific target, recombinant CIfB protein (specific binding)
   The binding of the peptide to beads coated with bovine serum albumin, BSA (unspecific binding)
   The binding of the peptide to beads coated with recombinant gluthatione S-transferase protein (unspecific binding)
○ A peptide corresponding to the binding-peptide was synthesized and tested for specific binding to the target protein addressed in the PPI and tested for inhibition of the PPI by competition. The tests were performed with the synthetic peptide, called IPEP-SA21, and two recombinant target proteins, namely recombinant Frag2-CDS and recombinant CIfB, using in vitro pull-down competition analysis and far-western affinity analysis.

With the above approach, peptide binders of defined target proteins, exemplified by the bacterial adhesins, can be identified and can be subjected to modification and subsequent selection using the Y2H system. Therefore, the approach can be extended to other components of the host-pathogen interaction and even beyond that to interacting proteins within the bacterium or within the host cell, which are implicated in establishing a successful infection and colonization of the host. As the approach is of a general nature, it can be used to any PPI in any system as a method to identify peptides binding to defined proteins.

### Example 1: Initial Y2H-screenings with S. aureus virulence factors

### 1.1. Primary Y2H-PPI hits

A Y2H-screen with the virulence factors of ***S. aureus*** (clumping factor b (CIfB)), fibronectin binding protein (Fnbp), Elastin binding protein (EbpS), and Collagen binding protein (CNA) was initiated.

Two different Y2H-screening libraries were used for this purpose, a genomic library of S. aureus and a cDNA library of human keratinocytes. Both libraries were house-made by general library cloning strategies.

CIfB and FnbB were screened against the human cDNA library, whereas EbpS and CNA were screened against the S. aureus genomic library.

Each screen was performed separately and resulted in "positive Y2H colonies". Positive Y2H colonies are yeast colonies, which contain a pair of plasmids encoding for proteins or protein fragments that can interact with each other. Interacting pairs of proteins or protein fragments can turn on a reporter gene. The reporter gene is a gene, which is translated into a protein (e.g. HIS3 protein product), which enables a yeast cell deficient in a distinct essential gene (e.g. HIS3, ADE2) to grow on yeast media lacking a substance e.g. the amino acid histidine, or adenine.

The Y2H-positive colonies were lysed, and the plasmids responsible for interaction were isolated, amplified and re-introduced in a Y2H reporter strain. The re-introduction is done for re-production of the results in order to exclude artefactual activation of the reporter genes (e.g. genomic mutations that enable cell growth without PPI).

Plasmids from re-produced Y2H-positive colonies were sequenced for the identification of the gene translated in yeast to a protein capable in the interaction with the virulence factors. Protein- and gene-identification is done by using blast-alignment/search tool.
http://www.ncbi.nlm.nih.gov/BLAST/
The screenings delivered PPIs, which were not publicly known to interact with the virulence factors CIfB, FnbB, EbpS or CNA. A summary of the genes encoding for PPI-partners can be seen in Table 1

**Table 1 Interaction partners detected by Y2H screening in example 1.1.**

| **Virulence factor** | **NCBI Protein GI** | **Interacting protein name** | **NCBI Protein GI** |
|---|---|---|---|
| FnbB | 15925492 | CGI-128 protein | 7706342 |
| | | HSPC118 | 6841458 |
| FnbB | 15925492 | Koyt binding protein 3 | 20149227 |
| FnbB | 15925492 | MT1E protein | 16307221 |
| FnbB | 15925492 | RING1 and YY1 binding protein | 22209026 |
| | | apoptin-associating protein 1 | 29423711 |
| | | death effector domain-associated factor | 5802964 |
| FnbB | 15925492 | Lysosomal Sialidase NEU 1 | 30583251 |
| FnbB | 15925492 | ATF6 | |
| | | CAMP responsive element binding protein-like | 3953531 |
| | | | 50604104 |
| | | Put. DNA binding protein | 1359755 |
| FnbB | 15925492 | Breakpoint cluster region protein | 55741849 |
| FnbB | 15925492 | CCT-beta | 2559012 |
| FnbB | 15925492 | H2B histone family, member T | 55930912 |
| FnbB | 15925492 | heterogeneous nuclear ribonucleoprotein D | 51477708 |
| FnbB | 15925492 | Hypothetical protein | 6808254 |
| FnbB | 15925492 | Neurexin III-alpha | 3800892 |
| FnbB | 15925492 | Ribosomal protein L13 | 15341812 |
| FnbB | 15925492 | Squalene monooxygenase | 16877565 |
| FnbB | 15925492 | SR rich protein | 18642526 |
| FnbB | 15925492 | UBC protein | 38197157 |
| FnbB | 15925492 | Ubiquitin B, precursor | 15929389 |
| FnbB | 15925492 | Armadillo repeat containing, X-linked 3 | 13528786 |
| FnbB | 15925492 | beta - actin | 15928803 |
| FnbB | 15925492 | CTCL tumor antigen HD-CL-08 | 22854566 |
| FnbB | 15925492 | DNAJA3 protein | 15706432 |
| FnbB | 15925492 | E2F transcription factor 6 variant | 33383323 |
| FnbB | 15925492 | FLNA protein | 15779184 |
| FnbB | 15925492 | Forkhead-associated domain histidinetriad like protein | 32394378 |
| | | Aprataxin isoform b | 28329430 |
| FnbB | 15925492 | hypothetical protein LOC51647 | 7706343 |
| FnbB | 15925492 | KIAA0319L | 14017891 |
| FnbB | 15925492 | PNAS-110 | 10834776 |
| FnbB | 15925492 | ATP synthase subunit c precursor | 285908 |
| FnbB | 15925492 | Hypothetical protein | 5541863 |
| FnbB | 15925492 | Neoplasm-related C140 product | 546831 |
| FnbB | 15925492 | Non-muscle myosin light chain | 189020 |
| FnbB | 15925492 | PD2 protein | 12054502 |
| FnbB | 15925492 | Ribosomal protein L23 | 38571606 |
| FnbB | 15925492 | Ribosomal protein S20 | 3088340 |
| FnbB | 15925492 | Ribosomal protein S20 | 4506697 |
| FnbB | 15925492 | RIE2 sid2705 | 5931614 |
| FnbB | 15925492 | SR rich protein | 18642528 |
| FnbB | 15925492 | | 5453603 |
| | | Homo sapiens chaperonin containing TCP1, subunit 2 (beta) (CCT2) | |
| FnbB | 15925492 | | 13027602 |
| | | hypothetical protein LOC65992 | |
| FnbB | 15925492 | | 33359221 |
| | | polycystic kidney disease 1-like isoform a | |
| CIfB | 15925620 | KRT8 | 50368987 |
| CIfB | 15925620 | KRT5 | 119395754 |
| CIfB | 15925620 | KRT6A | 15559584 |
| Cna | 387880 | DnaJ | 62900221 |
| Ebps | 15487782 | Conserved hypothetical protein | 49243011 |
| Ebps | 15487782 | Aminoacyltransferase femX | 81650573 |

### 1.2. Evaluation and confirmation of the quality of identified PPIs

The candidate PPI-partners were analysed within the Y2H-system to determine whether a) the correct reading frame of the gene is maintained, b) the orientation of the gene insert is correct, c) the portion of the gene insert corresponds to gene-coding regions (e.g. 5-primed or 3-primed regions so-called untranslated regions are not relevant for proteins), d) genomic regions, which do not correspond to annotated genes so far.

For elimination of one distinct class of Y2H false-positives (spurious activation of the reporter system within a Y2H-system that leads to the arising of Y2H-positive colonies without an interaction, e.g. false conclusion due to proteins interacting with the transcriptional apparatus of the yeast cells) a confirming experiment was performed. The best method for the elimination of a Y2H false-positive is to use a technique that depends on a completely different mechanism (e.g. confirming a molecular genetic result by a biochemical approach, e.g. confirming Y2H-results by a so-called pull-down approach or immuneprecipitation experiment). This procedure enables the elimination of one class of Y2H-false positives, the class of "technical" false positives. Recombinant proteins were produced from the isolated plasmids from the PPI-partners in a rabbit reticulocyte-based translation system and additionally in E.coli cells. The produced recombinant proteins were used for pull-down and immuneprecipitation experiments. The PPIs confirmed by these techniques are listed in Table 2. Figure 1 shows a pull-down experiment confirmation of PPI-partners from FnbB. Figure 2 shows a pull-down experiment, confirming the interaction between CIfB and KRT8

**Table 2: PPI confirmed according to experiments in example 1.2.**

| **Virulence factor** | **NCBI Protein GI** | **Interacting protein name** | **NCBI Protein GI** |
|---|---|---|---|
| FnbB | 15925492 | CGI-128 protein | 7706342 |
| | | HSPC118 | 6841458 |
| FnbB | 15925492 | Lysosomal Sialidase NEU 1 | 30583251 |
| FnbB | 15925492 | CCT-beta | 2559012 |
| FnbB | 15925492 | beta - actin | 15928803 |
| FnbB | 15925492 | FLNA protein | 15779184 |
| FnbB | 15925492 | CGI-128 protein | 7706342 |
| | | HSPC118 | 6841458 |
| CIfB | 15925620 | KRT8 | 50368987 |
| CIfB | 15925620 | KRT5 | 119395754 |
| CIfB | 15925620 | KRT6A | 15559584 |

### 1.3 Characterization of the interacting proteins and amino acid sequences sufficient for an interaction in the Y2H-system.

A Y2H-system is based on the interaction of two polypeptides, which can be either complete proteins (encoded by a full-length gene), domains of a protein or even fragments of a protein corresponding to peptides. Standard cDNA-libraries and genomic libraries used for Y2H-screening contain a mixture of nucleic acid sequences, ranging from complete full-length genes to gene-fragments encoding for a small portion of a protein. This diversity is caused by the construction procedure itself, which is a technical limitation of library constructions in general. Thus, a Y2H positive yeast colony can arise from interactions between proteins or fragments thereof (e.g. Protein-Protein, Protein-Protein domain, Protein-Peptide, Protein domain-Peptide etc.). The amino acid constituents of the interacting molecules were identified by comparing the sequence results of the isolated plasmids encoding for the identified PPI-partners with the sequence of the complete open reading frames of the full-length genes annotated in Genbank (http)://www.ncbi.nlm.nih.gov/). Table 3 shows the full-length amino acid sequences of proteins used and identified in this Y2H-experiment. The amino acid sequences sufficient for an interaction within the Y2H-system are extracted and listed in Table 3.

**Table 3: Full-length amino acid sequences of proteins used and identified in the Y2H-experiment of example 1.3. Amino acid sequence sufficient for interaction is underlined**

| **Protein NCBI** | **Protein sequence of the interactor** |
|---|---|
| | |
| **SEQ-ID and protein name of the virulence factor** | |
| **15925492** fibronectin-binding protein homolog [Staphylococcus aureus] | MVGGGGVGGGLLENANPLIYQRSGERPVTAGEEDEQVPDSIDAREIFDLIRSINDPE HPLTLEELNVVEQVRVQVSDPESTVAVAFTPTIPHCSMATLIGLSIKVKLLRSLPQRF KMDVHITPGTHASEHAVNKQLADKERVAAALENTHLLEVVNQCLSARS |
| **15925492 fibronectin-binding protein homolog [Staphylococcus aureus**] | MDNCLAAAALNGVDRRSLQRSARLALEVLERAKRRAVDWHALERPKGCMGVLARE APHLEKQPAAGPQRVLPGEKYYSSVPEEGGATHVYRYHRGESKLHMCLDIGNGQR KDRKKTSLGPGGSYQISEHAPEASQPAENISKDLYIEVYPGTYSVTVGSNDLTKKTH VVAVDSGQSVDLVFPV |
| **15925492** fibronectin-binding protein homolog [Staphylococcus aureus] | MDPNCSCATGGSCTCAGSCKCKECKCTSCKKSCCSCCPVGCAKCAQGCVCK- GASEKCSCCA |
| **15925492** fibronectin-binding protein homolog [Staphylococcus aureus] | MTMGDKKSPTRPKRQAKPATDEGFWDCSVCTFRNSAEAFKCSICDVRKGTSTRKP RINSQLVAQQVAQQYATPPPPKKEKKEKVEKQDKEKPEKDKEISPSVTKKNTNKKTK PKSDILKDPPSEANSIQSANATTKTSETNHTSRPRLKNVDRSTAQQLAVTVGNVTVII TDFKEKTRSSSTSSSTVTSSAGSEQQNQSSSGSESTDKGSSRSSTPKGDMSAV- NDESF |
| **15925492** fibronectin-binding protein homolog [Staphylococcus aureus] | |
| **15925492** | |
| fibronectin-binding protein homolog [Staphylococcus aureus] | |
| **15925492** fibronectin-binding protein homolog [Staphylococcus aureus] | |
| **15925492** fibronectin-binding protein homolog [Staphylococcus aureus] | MKRRAGLGGSMRSVVGFLSQRGLHGDPLLTQDFQRRRLRGCRNLYKKDLLGHFG CVNAIEFSNNGGQWLVSGGDDRRVLLWHMEQAIHSRVKPIQLKGEHHSNIFCLAFN SGNTKVFSGGNDEQVILHDVESSETLDVFAHEDAVYGLSVSPVNDNIFASSSDDGR VLIWDIRESPHGEPFCLANYPSAFHSVMFNPVEPRLLATANSKEGVGLWDIRKPQSS LLRYGGNLSLQSAMSVRFNSNGTQLLALRRRLPPVLYDIHSRLPVFQFDNQGYFNS CTMKSCCFAGDRDQYILSGSDDFNLYMWRIPADPEAGGIGRVVNGAFMVLKGHRSI VNQVRFNPHTYMICSSGVEKIIKIWSPYKQPGCTGDLDGRIEDDSRCLYTHEEYISLV LNSGSGLSHDYANQSVQEDPRMMAFFDSLVRREIEGWSSDSDSDLSESTILQLHAG VSERSGYTDSESSASLPRSPPPTVDESADNAFHLGPLRVTTTNTVASTPPTPTCED AASRQQRLSALRRYQDKRLLALSNESDSEENVCEVELDTDLFPRPRSPSPEDESSS SSSSSSSEDEEELNERRASTWQRNAMRRRQKTTREDKPSAPIKPTNTYIGEDNYDY PQIKVDDLSSSPTSSPERSTSTLEIQPSRASPTSDIESVERKIYKAYKWLRYSYISYSN NKDGETSLVTGEADEGRAGTSHKDNPAPSSSKEACLNIAMAQRNQDLPPEGCSKD TFKEETPRTPSNGPGHEHSSHAWAEVPEGTSQDTGNSGSVEHPFETKKLNGKALS SRAEEPPSPPVPKASGSTLNSGSGNCPRTQSDDSEERSLETICANHNNGRLHPRP PHPHNNGQNLGELEVVAYSSPGHSDTDRDNSSLTGTLLHKDCCGSEMACETPNAG |
| | TREDPTDTPATDSSRAVHGHSGLKRQRIELEDTDSENSSSEKKLKT |
| **15925492** fibronectin-binding protein homolog [Staphylococcus aureus] | MPEPAKSAPAPKKGSKKAVTKAQKKDGKKRKRSRKESYSVYVYKVLKQVHPDTGIS SKAMGIMNSFVNDIFERIAGEASRLAHYNKRSTITSREIQTAVRLLLPGELAKHAVSE GTKAVTKYTSAK |
| **15925492** fibronectin-binding protein homolog [Staphylococcus aureus] | MSEEQFGGDGAAAAATAAVGGSAGEQEGAMVAATQGAAAAAGSGAGTGGGTAS GGTEGGSAESEGAKIDASKNEEDEGKMFIGGLSWDTTKKDLKDYFSKFGEVVDCTL KLDPITGRSRGFGFVLFKESESVDKVMDQKEHKLNGKVIDPKRAKAMKTKEPVKKIF VGGLSPDTPEEKIREYFGGFGEVESIELPMDNKTNKRRGFCFITFKEEEPVKKIMEK KYHNVGLSKCEIKVAMSKEQYQQQQQWGSRGGFAGRARGRGGDQQSGYGKVSR RGGHQNSYKPY |
| **15925492** fibronectin-binding protein homolog [Staphylococcus aureus] | EAEEAPGARPQLQDAWRGPREPGPAGRGDGDSGRSQREGQGEGETQEAAAAAR RQEQTLRDATMEVQRGQFQGRPVSVWDVLFSSYLSEAHRDELLAQHAAGALGLPD LVAVLTRVIEETEERLSKVSFRGLRRQVSASELHTSGILGPETLRDLAQGTKTLQEVT EMDSVKRYLEGTSCIAGVLVPAKDQPGRQEKMSIYQAMWKGVLRPGTALVLLEAQ AATGFVIDPVRNLRLSVEEAVAAGVVGGEIQEKLLSAERAVTGYTDPYTGQQISLFQ AMQKDLIVREHGIRLLEAQIATGGVIDPVHSHRVPVDVAYRRGYFDEEMNRVLADPS DDTKGFFDPNTHENLTYLQLLQRATLDPETGLLFLSLSLQ |
| **15925492** fibronectin-binding protein homolog [Staphylococcus aureus] | MSSTLHSVFFTLKVSILLGSLLGLCLGLEFMGLPNQWARYLRWDASTRSDLSFQFKT NVSTGLLLYLDDGGVCDFLCLSLVDGRVQLRFSMDCAETAVLSNKQVNDSSWHFL MVSRDRLRTVLMLDGEGQSGELQPQRPYMDVVSDLFLGGVPTDIRPSALTLDGVQ AMPGFKGLILDLKYGNSEPRLLGSRGVQMDAEGPCGERPCENGGICFLLDGHPTC DCSTTGYGGKLCSEDVSQDPGLSHLMMSEQ |
| **15925492** fibronectin-binding protein homolog [Staphylococcus aureus] | |
| **15925492** fibronectin-binding protein homolog [Staphylococcus aureus] | MWDQGGQPWQQWPLNQQQWMQSFQHQQDPSQIDWAALAQAWIAQREASGQQ SMVEQPPGMMPNGQDMSTMESGPNNHGNFQGDSNFNRMWQPEWGMHQQPPH PPPDQPWMPPTPGPMDIVPPSEDSNSQDSGEFAPDNRHIFNQNNHNFGGPPDNF AVGPVNQFDYQHGAAFGPPQGGFHPPYWQPGPPGPPAPPQNRRERPSSFRDRQ RSPIALPVKQEPPQIDAVKRRTLPAWIREGLEKMEREKQKKLEKERMEQQRSQLSK KEKKATEDAEGGDGPRLPQRSKFDSDEEEEDTENVEAASSGKVTRSPSPVPQEEH SDPEMTEEEKEYQMMLLTKMLLTEILLDVTDEEIYYVAKDAHRKATKAPAKQLAQSS |
| | ALASLTGLGGLGGYGSGDSEDERSDRGSESSDTDDEELRHRIRQKQEAFWRKEKE QQLLHDKQMEEEKQQTERVTKEMNEFIHKEQNSLSLLEAREADGDVVNEKKRTPN ETTSVLEPKKEHKEKEKQGRSRSGSSSSGSPSSNSRTSSTSSTVSSSSYSSSSGSS RTSSRSSSPKRKKRHSRSRSPTIKARRSRSRSYSRRIKIESNRARVKIRDRRRSNRN SIERERRRNRSPSRERRRSRSRSRDRRTNRASRSRSRDRRKIDDQRGNLSGNSHK HKGEAKEQERKKERSRSIDKDRKKKDKEREREQDKRKEKQKREEKDFKFSSQDDR LKRKRESERTFSRSGSISVKIIRHDSRQDSKKSTTKDSKKHSGSDSSGRSSSESPGS SKEKKAKKPKHSRSRSAEKSQRSGKKASRKHKSKSRSR |
| **15925492** fibronectin-binding protein homolog [Staphylococcus aureus] | GFGSRFLFVDRCDRHLTMQIFVKTLTGKTITLEVEPSDTIENVKAKIQDKEGIPPDQQ RLIFAGKQLEDGRTLSDYNIQKESTLHLVLRLRGGMQIFVKTLTGKTITLEVEPSDTIE NVKAKIQDKEGIPPDQQRLIFAGKQLEDGRTLSDYNIQKESTLHLVLRLRGGMQIFVK TLTGKTITLEVEPSDTIENVKAKIQDKEGIPPDQQRLIFAGKQLEDGRTLSDYNIQKES TLHLVLRLRGGMQIFVKTLTGKTITLEVEPSDTIENVKAKIQDKEGIPPDQQRLIFAGK QLEDGRTLSDYNIQKESTLHLVLRLRGGMQIFVKTLTGKTITLEVEPSDTIENVKAKIQ DKEGIPPDQQRLIFAGKQLEDGRTLSDYNIQKESTLHLVLRLRGGMQIFVKTLTGKTI TLEVEPSDTIENVKAKIQDKEGIPPDQQRLIFAGKQLEDGRTLSDYNIQKESTLHLVLR LRGGMQIFVKTLTGKTITLEVEPSDTIENVKAKIQDKEGIPPDQQRLIFAGKQLEDGRT LSDYNIQKESTLHLVLRLRGGMQIFVKTLTGKTITLEVEPSDTIENVKAKIQDKEGIPP DQQRLIFAGKQLEDGRTLSDYNIQKESTLHLVLRLRGGMQIFVKTLTGKTITLEVEPS DTIENVKAKIQDKEGIPPDQQRLIFAGKQLEDGRTLSDYNIQKESTLHLVLRLRGGV |
| **15925492** fibronectin-binding protein homolog [Staphylococcus aureus] | MGYARKVGWVTAGLVIGAGACYCIYRLTRGRKQNKEKMAEGGSGDVDDAGDCSG ARYNDWSDDDDDSNESKSIVWYPPWARIGTEAGTRARARARARATRARRAVQKR ASPNSDDTVLSPQELQKVLCLVEMSEKPYILEAALIALGNNAAYAFNRDIIRDLGGLPI VAKILNTRDPIVKEKALIVLNNLSVNAENQRRLKVYMNQVCDDTITSRLNSSVQLAGL RLLTNMTVTNEYQHMLANSISDFFRLFSAGNEETKLQVLKLLLNLAENPAMTRELLR AQVPSSLGSLFNKKENKEVILKLLVIFENINDNFKWEENEPTQNQFGEGSLFFFLKEF QVCADKVLGIESHHDFLVKVKVGKFMAKLAEHMFPKSQE |
| **15925492** fibronectin-binding protein homolog [Staphylococcus aureus] | MQIFVKTLTGKTITLEVEPSDTIENVKAKIQDKEGIPPDQQRLIFAGKQLEDGRTLSDY NIQKESTLHLVLRLRGGMQIFVKTLTGKTITLEVEPSDTIENVKAKIQDKEGIPPDQQR LIFAGKQLEDGRTLSDYNIQKESTLHLVLRLRGGMQIFVKTLTGKTITLEVEPSDTIEN VKAKIQDKEGIPPDQQRLIFAGKQLEDGRTLSDYNIQKESTLHLVLRLRGGC |
| **15925492** fibronectin-binding protein homolog [Staphylococcus aureus] | MDDDIAALVVDNGSGMCKAGFAGDDAPRAVFPSIVGRPRHQGVMVGMGQKDSYV GDEAQSKRGILTLKYPIEHGIVTNWDDMEKIWHHTFYNELRVAPEEHPVLLTEAPLN PKANREKMTQIMFETFNTPAMYVAIQAVLSLYASGRTTGIVMDSGDGVTHTVPIYEG YALPHAILRLDLAGRDLTDYLMKILTERGYSFTTTAEREIVRDIKEKLCYVALDFEQEM ATAASSSSLEKSYELPDGQVITIGNERFRCPEALFQPSFLGMESCGIHETTFNSIMKC DVDIRKDLYANTVLSGGTTMYPGIADRMQKEITALAPSTMKIKIIAPPERKYSVWIGG SILASLSTFQQMWISKQEYDESGPSIVHRKCF |
| **15925492** fibronectin-binding protein homolog [Staphylococcus aureus] | MITGTSQADCAVLIVAAGVGEFEAGISKNGQTREHALLAYTLGVKQLIVGVNKMDST EPPYSQKRYEEIVKEVSTYIKKIGYNPDTVAFVPISGWNGDNMLEPSANMPWFKGW KVTRKDGNASGTTLLEALDCILPPTRPTDKPLRLPLQDVYKIGGIGTVPVGRVETGVL KPGMVVTFAPVNVTTEVKSVEMHHEALSEALPGDNVGFNVKNVSVKDVRRGNVAG DSKNDPPMEAAGFTAQVIILNHPGQISAGYAPVLDCHTAHIACKFAELKEKIDRRSGK KLEDGPKFLKSGDAAIVDMVPGKPMCVESFSDYPPLGRFAVRDMRQTVAVGVIKAV DKKAAGAGKVTKSAQKAQKAK |
| **15925492** fibronectin-binding protein homolog [Staphylococcus aureus] | MELTFNQAAKGVNKEFTVNIMDTCERCNGKGNEPGTKVQHCHYCGGSGMETINTG PFVMRSTCRRCGGRGSIIISPCVVCRGAGQAKQKKRVMIPVPAGVEDGQTVRMPV GKREIFITFRVQKSPVFRRDGADIHSDLFISIAQALLGGTARAQGLYETINVTIPPGTQ TDQKIRMGGKGIPRINSYGYGDHYIHIKIRVPKRLTSRQQSLILSYAEDETDVEGTVN GVTLTSSGKRSTGN |
| **15925492** fibronectin-binding protein homolog [Staphylococcus aureus] | MEKRLGVKPNPASWILSGYYWQTSAKWLRSLYLFYTCFCFSVLWLSTDASESRCQ QGKTQFGVGLRSGGENHLWLLEGTPSLQSCWAACCQDSACHVFWWLEGMCIQA DCSRPQSCRAFRTHSSNSMLVFLKKFQTADDLGFLPEDDVPHLLGLGWNWASWR QSPPRAALRPAVSSSDQQSLIRKLQKRGSPSDVVTPIVTQHSKVNDSNELGGLTTS GSAEVHKAITISSPLTTDLTAELSGGPKNVSVQPEISEGLATTPSTQQVKSSEKTQIA VPQPVAPSYSYATPTPQASFQSTSAPYPVIKELVVSAGESVQITLPKNEVQLNAYVL QEPPKGETYTYDWQLITHPRDYSGEMEGKHSQILKLSKLTPGLYEFKVIVEGQNAH GEGYVNVTVKPEPRKNRPPIAIVSPQFQEISLPTTSTVIDGSQSTDDDKIVQYHWEEL KGPLREEKISEDTAILKLSKLVPGNYTFSLTVVDSDGATNSTTANLTVNKAVDYPPVA NAGPNQVITLPQNSITLFGNQSTDDHGITSYEWSLSPSSKGKVVEMQGVRTPTLQL SAMQEGDYTYQLTVTDTIGQQATAQVTVIVQPENNKPPQADAGPDKELTLPVDSTT LDGSKSSDDQKIISYLWEKTQGPDGVQLENANSSVATVTGLQVGTYVFTLTVKDER NLQSQSSVNVIVKEEINKPPIAKITGNVVITLPTSTAELDGSKSSDDKGIVSYLWTRDE GSPAAGEVLNHSDHHPILFLSNLVEGTYTFHLKVTDAKGESDTDRTTVEVKPDPRKN NLVEIILDINVSQLTERLKGMFIRQIGVLLGVLDSDIIVQKIQPYTEQSTKMVFFVQNEP PHQIFKGHEVAAMLKSELRKQKADFLIFRALEVNTVTCQLNCSDHGHCDSFTKRCIC DPFWMENFIKVQLRDGDSNCEWSVLYVIIATFVIVVALGILSWTVICCCKRQKGKPKR KSKYKILDATDQESLELKPTSRAGIKQKGLLLSSSLMHSESELDSDDAIFTWPDREK GKLLHGQNGSVPNGQTPLKARSPREEIL |
| **15925492** fibronectin-binding protein homolog [Staphylococcus aureus] | MDLVRSAPGGILDLNKVATKLGVRKRRVYDITNVLDGIDLVEKKSKNHIRWIGSDLSN FGAVPQQKKLQEELSDLSAMEDALDELIKDCAQQLFELTDDKENERLAYVTYQDIHS IQAFHEQIVIAVKAPAETRLDVPAPREDSITVHIRSTNGPIDVYLCEVEQGQTSNKRSE GVGTSSSESTHPEGPEEEENPQQSEELLEVSN |
| **15925492** fibronectin- | MPSGKVAQPTITDNKDGTVTVRYAPSEAGLHEMDIRYDNMHIPGSPLQFYVDYVNC GHVTAYGPGLTHGVVNKPATFTVNTKDAGEGGLSLAIEGPSKAEISCTDNQDGTCS |
| binding protein homolog [Staphylococcus aureus] | VSYLPVLPGDYSILVKYNEQHVPGSPFTARVTGDDSMRMSHLKVGSAADIPINISET DLSLLTATVVPPSGREEPCLLKRLRNGHVGISFVPKETGEHLVHVKKNGQHVASSPI PVVISQSEIGDASRVRVSGQGLHEGHTFEPAEFIIDTRDAGYGGLSLSIEGPSKVDIN TEDLEDGTCRVTYCPTEPGNYIINIKFADQHVPGSPFSVKVTGEGRVKESITRRRRA PSVANVGSHCDLSLKIPEISIQDMTAQVTSPSGKTHEAEIVEGENHTYCIRFVPAEMG THTVSVKYKGQHVPGSPFQFTVGPLGEGGAHKVRAGGPGLERAEAGVPAEFSIWT REAGAGGLAIAVEGPSKAEISFEDRKDGSCGVAYVVQEPGDYEVSVKFNEEHIPDS PFVVPVASPSGDARRLTVSSLQESGLKVNQPASFAVSLNGAKGAIDAKVHSPSGAL EECYVTEIDQDKYAVRFIPRENGVYLIDVKFNGTHIPGSPFKIRVGEPGHGGDPGLV SAYGAGLEGGVTGNPAEFVVNTSNAGAGALSVTIDGPSKVKMDCQECPEGYRVTY TPMAPGSYLISIKYGGPYHIGGSPFKAKVTGPRLVSNHSLHETSSVFVDSLTKATCA PQHGAPGPGPADASKVVAKGLGLSKAYVGQKSSFTVDCSKAGNNMLLVGVHGPR TPCEEILVKHVGSRLYSVSYLLKDKGEYTLVVKWGDEHIPGSPYRVVVP |
| **15925492** fibronectin-binding protein homolog [Staphylococcus aureus] | MSNVNLSVSDFWRVMMRVCWLVRQDSRHQRIRLPHLEAVVIGRGPETKITDKKCS RQQVQLKAECNKGYVKVKQVGVNPTSIDSVVIGKDQEVKLQPGQVLHMVNELYPYI VEFEEEAKNPGLETHRKRKRSGNSDSIERDAAQEAEAGTGLEPGSNSGQCSVPLK KGKDAPIKKESLGHWSQGLKISMQDPKMQVYKDEQVVVIKDKYPKARYHWLVLPW TSISSLKAVAREHLELLKHMHTVGEKVIVDFAGSSKLRFRLGYHAIPSMSHVHLHVIS QDFDSPCLKNKKHWNSFNTEYFLESQAVIEMVQEAGRVTVRDGMPELLKLPLRCH ECQQLLPSIPQLKEHLRKHWTQ |
| **15925492** fibronectin-binding protein homolog [Staphylococcus aureus] | MSNVNLSVSDFWRVMMRVCWLVRQDSRHQRIRLPHLEAVVIGRGPETKITDKKCS RQQVQLKAECNKGYVKVKQVGVNPTSIDSVVIGKDQEVKLQPGQVLHMVNELYPYI VEFEEEAKNPGLETHRKRKRSGNSDSIERDAAQEAEAGTGLEPGSNSGQCSVPLK KGKDAPIKKESLGHWSQGLKISMQDPKMQVYKDEQVVVIKDKYPKARYHWLVLPW TSISSLKAVAREHLELLKHMHTVGEKVIVDFAGSSKLRFRLGYHAIPSMSHVHLHVIS QDFDSPCLKNKKHWNSFNTEYFLESQE |
| **15925492** fibronectin-binding protein homolog [Staphylococcus aureus] | VMEGKHSQILKLSKLTPGLYEFKVIVEGQNAHGEGYVNVTVKPEPRKNRPPIAIVSP QFQEISLPTTSTVIDGSQSTDDDKIVQYHWEELKGPLREEKISEDTAILKLSKLVPGN YTFSLTVVDSDGATNSTTANLTVNKAVDYPPVANAGPNQVITLPQNSITLFGNQSTD DHGITSYEWSLSPSSKGKVVEMQGVRTPTLQLSAMQEGDYTYQLTVTDTIGQQATA QVTVIVQPENNKPPQADAGPDKELTLPVDSTTLDGSKSSDDQKIISYLWEKTQGPD GVQLENANSSVATVTGLQVGTYVFTLTVKDERNLQSQSSVNVIVKEEINKPPIAKITG NVVITLPTSTAELDGSKSSDDKGIVSYLWTRDEGSPAAGEVLNHSDHHPILFLSNLVE GTYTFHLKVTDAKGESDTDRTTVEVKPDPRKNNLVEIILDINVSQLTERLKGMFIRQI GVLLGVLDSDIIVQKIQPYTEQSTKMVFFVQNEPPHQIFKGHEVAAMLKSELRKQKA DFLIFRALEVNTVTCQLNCSDHGHCDSFTKRCICDPFWMENFIKVQLRDGDSNCEW SVLYVIIATFVIVVALGILSWTVICCCKRQKGKPKRKSKYKILDATDQESLELKPTSRA GRGPGCQSF |
| **15925492** fibronectin- | MNARGLGSELKDSIPVTELSASGPFESHDLLRKGFSCVKNELLPSHPLELSEKNFQL NQDKMNFSTLRNIQGLFAPLKLQMEFKAVQQVQRLPFLSSSNLSLDVLRGNDETIGF |
| binding protein homolog [Staphylococcus aureus] | EDILNDPSQSEVMGEPHLMVEYKLGYCNSVLFMETEGCILFIVIFVL |
| **15925492** fibronectin-binding protein homolog [Staphylococcus aureus] | MASLSLAPVNIFKAGADEERAETARLTSFIGAIAIGDLVKSTLGPKGMDKILLSSGRDA SLMVTNDGATILKNIGVDNPAAKVLVDMSRVQDDEVGDGTTSVTVLAAELLREAESL IAKKIHPQTIIAGWREATKAAREALLSSAVDHGSDEVKFRQDLMNIAGTTLSSKLLTH HKDHFTKLAVEAVLRLKGSGNLEAIHIIKKLGGSLADSYLDEGFLLDKKIGVNQPKRIE NAKILIANTGMDTDKIKIFGSRVRVDSTAKVAEIEHAEKEKMKEKVERILKHGINCFINR QLIYNYPEQLFGAAGVMAIEHADFAGVERLALVTGGEIASTFDHPELVKLGSCKLIEE VMIGEDKLIHFSGVALGEACTIVLRGATQQILDEAERSLHDALCVLAQTVKDSRTVYG GGCSEMLMAHAVTQLANRTPGKEAVAMESYAKALRMLPTIIADNAGYDSADLVAQL RAAHSEGNTTAGLDMREGTIGDMAILGITESFQVKRQVLLSAAEAAEVILRVDNIIKAA PRKRVPDHHPC |
| **15925492** fibronectin-binding protein homolog [Staphylococcus aureus] | MQTAGALFISPALIRCCTRGLIRPVSASFLNSPVNSSKQPSYSNFPLQVARREFQTS VVSRDIDTAAKFIGAGAATVGVAGSGAGIGTVFGSLIIGYARNPSLKQQLFSYAILGFA LSEAMGLFCLMVAFLILFAM |
| **15925492** fibronectin-binding protein homolog [Staphylococcus aureus] | MYSRKAMYKRKYSAAKSKVEKKKKEKVLATVTKPVGGDKNGGTRVVKLRKMPRYY PTEDVPRKLLSHGKKPFSQHVRKLRASITPGTILIILTGRHRGKRVVFLKQLASGLLLV TGPLVSIEFLYEEHTRNLSLPLQPKSISAIVKIPKHLTDAYFKKKKLRKPRHQEGEIFDT EKEKYEITEQRKIDQKLWTHKFYQKSKLFLSSS |
| **15925492** fibronectin-binding protein homolog [Staphylococcus aureus] | MRALGQNPTNAEVLKVLGNPKSDEMNVKVLDFEHFLPMLQTVAKNKDQGTYEDYV EGLRVFDKEGNGTVMGAEIRHVLVTLGEKMTEEEVEMLVAGHEDSNGCINY- EELVRMVLNG |
| **15925492** fibronectin-binding protein homolog [Staphylococcus aureus] | MAPTIQTQAQREDGHRPNSHRTLPERSGVVCRVKYCNSLPDIPFDPKFITYPFDQN RFVQYKATSLEKQHKHDLLTEPDLGVTIDLINPDTYRIDPNVLLDPADEKLLEEEIQAP TSSKRSQQHAKVVPWMRKTEYISTEFNRYGISNEKPEVKIGVSVKQQFTEEEIYKDR DSQITAIEKTFEDAQKSISQHYSKPRVTPVEVMPVFPDFKMWINPCAQVIFDSDPAP KDTSGAAALEMMSQAMIRGMMDEEGNQFVAYFLPVEETLKKRKRDQEEEMDYAP DDVYDYKIAREYNWNVKNKASKGYEENYFFIFREGDGVYYNELETRVRLSKRRAKA |
| | GVQSGTNALLVVKHRDMNEKELEAQEARKAQLENHEPEEEEEEMETEEKEAGGS DEEQEKGSSSEKEGSEDEHSGSESEREEGDRDEASDKSGSGEDESSEDEARAAR DKEEIFGSDADSEDDADSDDEDRGQAQGGSDNDSDSGSNGGGQRSRSHSRSAS PFPSGSEHSAQEDGSEAAASDSSEADSDSD |
| **15925492** fibronectin-binding protein homolog [Staphylococcus aureus] | MSKRGRGGSSGAKFRISLGLPVGAVINCADNTGAKNLYIISVKGIKGRLNRLPAAGV GDMVMATVKKGKPELRKKVRPAVVIRQRKSYRRKDGVFLYFEDNAGVIVNNKGEM KGSAITGPVAKECADLWPRIASNAGSIA |
| **15925492** fibronectin-binding protein homolog [Staphylococcus aureus] | MVAPVWYLVAAALLVGFILFLTRSRGRAASAGQEPLHNEELAGAGRVAQPGLEPE EPRAGGRPRRRRDLGSRLQAQRRAQRVAWAEADENEEEAVILAQEEEGVEKPAET HLSGKIGAKKLRKLEEKQARKAQREAEEAEREERKRLESQREAEWKKEEERLRLEE EQKEEEERKAREEQAQREHEEYLKLKEAFVVEEEGVGETMTEEQSQSFLTEFINYIK QSKVVLLEDLASQVGLRTQDTINRIQDLLAEGTITGVIDDRGKFIYITPEELAAVANFIR QRGRVSIAELAQASNSLIAWGRESPAQAPA |
| **15925492** fibronectin-binding protein homolog [Staphylococcus aureus] | KGPVRMPTKTLRITTRKTPCGEGSKTWDRFQMRIHKRLIDLHSPSEIVKQIT- SISIEPGV |
| **15925492** fibronectin-binding protein homolog [Staphylococcus aureus] | |
| **15925492** fibronectin-binding protein homolog [Staphylococcus aureus] | MPLSSPNAAATASDMDKNSGSNSSSASSGSSKGQQPPRSASAGPAGESKPKSDG KNSSGSKRYNRKRELSYPKNESFNNQSRRSSSQKSKTFNKMPPQRGGGSSKLFSS SFNGGRRDEVAEAQRAEFSPAQFSGPKKINLNHLLNFTFEPRGQTGHFEGSGHGS WGKRNKWGHKPFNKELFLQANCQFVVSEDQDYTAHFADPDTLVNWDFVEQVRIC SHEVPSCPICLYPPTAAKITRCGHIFCWACILHYLSLSEKTWSKCPICYSSVHKKDLK SVVATESHQYVVGDTITMQLMKREKGVLVALPKSKWMNVDHPIHLGDEQHSQYSK LLLASKEQVLHRVVLEEKVALEQQLAEEKHTPESCFIEAAIQELKTREEALSGLAGSR REVTGVVAALEQLVLMAPLAKESVFQPRKGVLEYLSAFDEETTEVCSLDTPSRPLAL PLVEEEEAVSEPEPEGLPEACDDLELADDNLKEGTICTESSQQEPITKSGFTRLSSS PCYYFYQAEDGQHMFLHPVNVRCLVREYGSLERSPEKISATVVEIAGYSMSEDVRQ |
| | RHRYLSHLPLTCEFSICELALQPPVVSKETLEMFSDDIEKRKRQRQKKAREERRRER RIEIEENKKQGKYPEVHIPLENLQQFPAFNSYTCSSDSALGPTSTEGHGALSISPLSR SPGSHADFLLTPLSPTASQGSPSFCVGSLEEDSPFPSFAQMLRVGKAKADVWPKTA PKKDENSLVPPAPVDSDGESDNSDRVPVPSFQNSFSQAIEAAFMKLDTPATSDPLS EEKGGKKRKKQKQKLLFSTSVVHTK |
| **15925492 fibronectin-binding protein homolog [Staphylococcus aureus]** | RTSSTSSTVSSSSYSSSSGSSRTSSRSSSPKRKKRHSRSRSPTIKARRSRSRSYSR RIKIESNRARVKIRDRRRSNRNSIERERRRNRSPSRERRRSRSRSRDRRTNRASRS RSRDRRKIDDQRGNLSGNSHKHKGEAKEQERKKERSRSIDKDRKKKDKEREREQD KRKEKQKREEKDFKFSSQDDRLKRKRESERTFSRSGSISVKIIRHDSRQDSKKSTTK DSKKHSGSDSSGRSSSESPGSSKEKKAKKPKHSRSRSAEKSQRSGKKASRKHK- SKSRSR |
| **15925620** Clumping factor B [Staphylococcus aureus subsp. aureus Mu50]. | MSIRVTQKSYKVSTSGPRAFSSRSYTSGPGSRISSSSFSRVGSSNFRGGLGGGYG GASGMGGITAVTVNQSLLSPLVLEVDPNIQAVRTQEKEQIKTLNNKFASFIDKVRFLE QQNKMLETKWSLLQQQKTARSNMDNMFESYINNLRRQLETLGQEKLKLEAELGNM QGLVEDFKNKYEDEINKRTEMENEFVLIKKDVDEAYMNKVELESRLEGLTDEINFLR QLYEEEIRELQSQISDTSVVLSMDNSRSLDMDSIIAEVKAQYEDIANRSRAEAESMY QIKYEELQSLAGKHGDDLRRTKTEISEMNRNISRLQAEIEGLKGQRASLEAAIADAEQ RGELAIKDANAKLSELEAALQRAKQDMARQLREYQELMNVKLALDIEIATYRKLLEG EESRLESGMQNMSIHTKTTSGYAGGLSSAYGGLTSPGLSYSLGSSFGSGAGSSSF SRTSSSRAVVVKKIETRDGKLVSESSDVLPK |
| **15925620** Clumping factor B [Staphylococcus aureus subsp. aureus Mu50]. | |
| **15925620** Clumping factor B [Staphylococcus aureus subsp. aureus Mu50]. | |
| | |
| **387880** collagen adhesin [Staphylococcus aureus] | MAKRDYYEVLGISKDASKDEIKKAYRKLSKKYHPDINKEEGADEKFKEISEAYEVLSD DNKRASYDQFGHDGPQGFGGQGFNGSDFGGFSGFGGGFDIFSSFFGGGRQRD PNAPQKGDDLQYTMTLTFEEAVFGTTKEISIRKDVTCETCHGDGAKPGTSKKTCSY CNGAGHVAVEQNTILGRVRTEQVCPKCNGSGQEFEEACPTCHGKGTENKTVKLEV KVPEGVDNEQQIRLAGEGSPGVNGGPAGDLYVVFRVKPSETFKRDGDDIYYKLNVS FPQAALGDEIKIPTLNNEVMLTIPAGTQTGKQFRLKEKGIKNVHGYGYGDLYVDIKVV TPTKLTDRQKELMKEFAQLNGEEINDQPSNFKDRAKRFFKGE |
| | |
| **15487782** cell surface elastin binding protein EbpS [Staphylococcus aureus] | MTNFTFDGAHSSLEFQIKHLMVSKVKGSFDQFDVAVEGDINDFSTLKATATIIPSSINT KNEARDNHLKSGDFFGTDEFDKITFVTKSITESKVVGDLTIKGITNEETFDVEFNGVS KNPMDGSQVTGIIVTGIINREKYGINFNQTLETGGVMLGKDVKFEASAEFSISE |
| **15487782** cell surface elastin binding protein EbpS [Staphylococcus aureus] | MEKMHITNQEHDAFVKSNPNGDLLQLTKWAETKKLTGWYARRIAVGRDGEIQGVA QLLFKKVPKLPYTLCYISRGFVVDYSNKEALNALLDSAKEIAKAEKAYAIKIDPDVEVD KGTDALQNLKALGFKHKGFKEGLSKDYIQPRMTMITPIDKNDDELLNSFERRNRSKV RLALKRGTTVERSDREGLKTFAELMKITGERDGFLTRDISYFENIYDALHEDGDAELF LVKLDPKENIAKVNQELNELHAEIAKWQQKMETSEKQAKKAQNMINDAQNKIAKNED LKRDLEALEKEHPEGIYLSGALLMFAGSKSYYLYGASSNEFRDFLPNHHMQYTMMK YAREHGATTYDFGGTDNDPDKDSEHYGLWAFKKVWGTYLSEKIGEFDYVLNQPLY QLIEQVKPRLTKAKIKISRKLKRK |

### Example 2: Construction of an ORFeome exemplified by the ORFeome of S. aureus

The sum of all open reading frames (ORFs) of all annotated genes from a given organism is called an ORFeome. By systematic cloning of an ORFeome one can construct a complete collection of genes for organism-wide research. The ORFeome is a resource. The construction of an ORFeome is a challenging undertaking, because each gene has to be amplified from a cDNA or genomic-DNA source with specific primers (e.g. for ***S.aureus*** about 6000 primers are needed) and has to be cloned one by one into plasmids. However, it enables a more comprehensive and systematic research because each gene's role (more precisely also each protein's role produced from a certain gene) is investigated in one experiment and no gene is skipped. Furthermore, there is no bias due to unequal representation of expressed genes as it is in cDNA-libraries (low abundant or high abundant expressed genes). If libraries are made from the ORFeome, one can construct libraries which are perfectly normalized for gene distribution and gene level. The ORFeome-resource is a collection of individual genes cloned into a plasmid and arranged in a micro-well plate format. From each ORF different samples are maintained for short- and long-term storage:
- Each ORF is maintained as pure plasmid DNA, which is PCR confirmed and sequenced
- Each ORF is maintained as a mixture of plasmid DNA, (the complete cloning reaction is transformed into E.coli and DNA is prepared from the mixture of ***E.coli*** cells)
- As an ***E.coli*** culture harbouring one type of plasmid encoding a specific ORF (contains no cloning artefacts, the inserted DNA is confirmed by PCR, the culture is homogenous)
- As an ***E.coli*** culture harbouring a mixture of plasmids encoding different cloning states of a specific ORF (contains also cloning artefacts e.g. empty plasmids, shortened or wrongly cloned nucleic acids, whereby the correct ORF represents an undefined part of the mixture)
- As a pure PCR-product of a specific ORF
- As a cloning mixture of each ORF from each cloning reaction

The ORFeome can be used either individually gene by gene, as a complete collection, subdivided into parts (partition by professional knowledge into sets of genes with similar molecular or biochemical functions according to gene-ontology criteria e.g. transcription factors, DNA-repair, glycolysis etc.) in a matrix format, as well as pooled into mixtures of ORF-containing plasmid-libraries. The libraries resulting from such pooling approaches are normalized for gene content and distribution and can be used for downstream applications e.g. Y2H-screening, gene-expression analysis, microarray analysis etc.

### 2.1. ORFeome design

- The complete genome sequence of the ***S. aureus*** strain Mu50 as a template for primer design was selected. Accession: NC 002758
- 2697 open reading frames of S.aureus gene were identified
- 2623 primers were designed and 2623 were used for cloning of the ORFeome.
- All primers used are designed for Gateway^{®}-compatibility, which means that any PCR-product generated by a pair of these primers can be used for recombinatorial cloning into vectors of the Gateway^{®}-system.
- The primers contain a sequence of a prokaryotic ribosome binding site, a sequence of an eukaryotic ribosome binding site (Kozak-consensus sequence), a sequence for annealing to the target gene (the gene that has to be amplified) and a sequence necessary for binding and extension by a second pair of universal primers. Universal primers are a forward and a reverse PCR-primer pair, which can extend any PCR-product with an additional stretch of a nucleic-acid sequence. This extension is necessary to function for Gateway^{®}-cloning. The two different ribosome binding sites (Shine Dalgarno and Kozak sequences) give one the flexibility to use the completed ORFeome for prokaryotic and eukaryotic expression independent from the composition of expression plasmids used in downstream applications. The extension of the PCR-product by the universal primers reassemble a nucleic acid sequence necessary for site-specific recombination principle of the Gateway^{®}-system.

### 2.2. ORFeome Cloning

- Genomic DNA from S. aureus was prepared from a clinical isolate.
- PCR reactions with the designed primers were done and resulting PCR-products were analysed by agarose gel electrophoresis. PCR-bands corresponding to the expected gene-size were used for recombinatorial cloning. This cloning step is called Gateway^{®} BP-cloning according to the manufacturer's manual.
- After the BP-cloning step, the DNA mixture is used to transform-E. coli cells (Top10 strain) and plated onto selection media containing antibiotics; positive E. coli colonies are confirmed by colony-PCR with vector specific primers. Only PCR-products corresponding to the estimated gene-length are counted as successfully cloned and are transferred to micro-well plates.
- From successfully cloned genes the following biomaterials are collected and archived for storage: All PCR-products stored in 96-well microplates, all E.coli cells harbouring a plasmid encoding for a distinct S. aureus gene collected in 96-well deepwell plates, all BP-reactions stored in 96-well microplates and all plasmids purified from E.coli cultures archived in 96-well microplates.
- Final confirmation of the cloning procedure is done by taking 300 random samples for DNA-sequencing. The sequencing results were positive; all control samples harboured the desired S. aureus gene. Table 5 gives an overview of all steps.

**Table 5: Overview of the ORFeome cloning steps**

| **Working step** | **Description** |
|---|---|
| Genomic template | Sequence retrieved from subspecies ***S. aureus*** Mu50. The complete genome is comprised of 2697 proteins (genes) |
| PCR-Primer designed | Primer designed for 2623 protein encoding genes |
| PCRs performed | PCRs for 2623 protein encoding genes have been performed |
| Cloning | 2623 cloning reactions performed |
| Confirmation by PCR | Cloning of 1750 genes into gateway compatible vectors could be confirmed by PCRs |
| Plasmid DNA isolation | Pure plasmid DNA was isolated from 2623 clones |
| Storage | PCR-products of each gene were frozen in 96-well plates Pure plasmid DNA was transferred into 96-well plates and frozen |
| | Living stocks of plasmid harbouring E.coli cultures were frozen in 96-deep well plates |
| Confirmation by DNA-sequencing | Sequencing of 400 cloned different S. aureus genes performed |

### Example 3: Libraries of pooled ORFeome

The aim was to construct a comprehensive and normalized Y2H-library, which contains all genes from an organism and is unique in composition
- Equal amounts of pure plasmids from each of the 2623 DNA-preparations were mixed each containing a specific ORF from ***S.aureus.*** See parts 6.1 and 6.2 for ORFeome cloning for the DNA-preparations used.
- The resulting pooled library is completely different from existing gene-libraries because the content of the existing genes is exactly known. Additionally, standard libraries are not guaranteed to contain complete genes; in fact they often contain gene fragments, non-coding regions such as 5-primed and 3-primed untranslated regions, and are to a large extend out-of frame for the gene.
- Additionally, we mixed equal amounts of living E.coli cultures each containing the 2623 different ***S. aureus*** ORFs.
- The titre of colony-forming units in the mixed E.coli culture stock was calculated and the volume was determined to put onto a E.coli culture plate to reach confluent colony growth over the complete surface of the plate.
- Measured aliquots of the "E.coli pool" were plated onto 500 petri-dishes (150 mm) containing media with antibiotics selective for the plasmid.
- After 12 hours, the E.coli colonies were scraped off the petri-dishes and pooled for a) subsequent plasmid preparation, and b) long-term liquid stocks, which can be used for re-amplification of the complete library at any time. This guarantees a constant quality of the library, without the risk to lose or to penalize a certain ORF.
- For quality control, competent E.coli cells were re-transformed with an aliquot of the plasmid preparation, resulting E.coli colonies were cultivated overnight, plasmids were isolated and the ORF it contained was sequenced. Each of the randomly taken 100 E.coli colonies contained a plasmid for a different ORF; no single ORF was found twice in the random-samples

### 3.1. Unspecific and unselected ORFomer-library

ORFomers are peptides derived from proteins encoded by the ORFeome. ORFomer libraries will be used to find binders for a certain protein target or group of target proteins. Thus, the functionality of the library will be determined by the feature to bind to (to have an affinity for) a protein or peptide. The complexity of an ORFomer library is comparable to random peptide libraries or antibody Fab-fragment libraries. However, ORFomers represent the diversity within all proteins in a given organism, not restricted to a single class of proteins (such as antibodies).

The S. aureus ORFeome was used for the construction of an unspecific and unselected ORFomer-library. The term unspecific means, that the library contains a broad spectrum of peptides from protein binders, but is not yet specific for a certain target. The term unselected means that the library has not been subjected to any selection or maturation steps (no iterative rounds of mutagenesis and selection). This type of ORFomer library is the basis for deriving all other ORFomer-library types, and is produced as follows:
- The ORFs from each plasmid of the ORFeome collection are amplified by PCR with specific primers binding to plasmid regions flanking the ORF, which is inserted in the Gateway-plasmid. 2623 PCR reactions were performed.
- PCR-products were measured for DNA-content by optical density measurement at 260 nm and the concentration of the amplified ORF is calculated. Equimolar ratios are taken from the complete reaction and mixed to get a pool of all ORFs.
- The DNA-mixture was fragmented into smaller pieces by sonification.
- The fragmented DNA was analysed by gel electrophoresis on standard agarose gels stained with ethidium-bromide.
- After electrophoresis, DNA migrating between 100 base pairs (bp) and 500 bp was excised and the DNA was extracted by using commercially available DNA-gele extraction kits.
- The DNA was end-repaired by T4-Polymerase and T4-polynucleotide kinase and ligated into Y2H prey-plasmids. The cloning was performed by blunt-end ligation into Smal restriction enzyme linearized plasmids with T4-ligase.
- The products of the cloning reactions were purified and transformed into chemically competent E.coli cells, and then plated onto selection-plates containing antibiotics.
- The titre of colony forming units of the cloning reaction was determined by transforming small aliquots onto plates. The volume of the cloning reaction, which results in a confluent growth on the complete surface of a plate containing E.coli media, was determined.
- A total of 2.5 million E.coli colonies were grown on 50 petri-dishes containing plasmid-selective media with the appropriate antibiotics, and used for plasmid DNA-isolation and long-term storage of E.coli cultures for re-amplification if required.
- Additionally, 30 million E.coli transformants were grown in liquid selection media. The total number of E.coli transformants reached a complexity of about 33 million clones in the final library. This guarantees, that each gene is allowed to be cloned and represented in ∼14,000 independent fragments (calculating with 2 kbp on average for S. aureus genes). This number is more than needed for many small-sized genes and there will be a bias towards smaller DNA-pieces for cloning into plasmids. However, considering the fact that 3 different reading frames, 2 different orientations, a heavy fragmentation degree, concatemerization of individual fragments at the ligation step and additional cloning biases can take place, the complexity makes sense and is justifiable for exhaustive screenings.
- The cloning efficiency was determined with control-PCR by taking random samples of E.coli colonies and detection of the cloned DNA-fragment. The primers used for this reaction bind to plasmid regions flanking the putative insert. The cloning efficiency was determined and shows >80% successfully cloned plasmids.
- This ORFomer library is suitable for any Y2H-system and can be used for the identification of peptides binding to one or more protein targets. This library contains the complete diversity of protein-binding scaffolds engineered by nature for the proteome of ***S.aureus.*** With ORFeomes from additional organisms, all protein-binding scaffolds of Nature can be made accessible.

### Example 4: Production of a specific peptide library from interacting proteins (specific for binding to one or more targets), but unselected ORFomer library

Exemplified by systematic mutagenesis: Shortening of the gene encoding the interacting protein, resulting in N- and C-terminal truncations, until the interaction is lost to identify the smallest peptide derived from the binding proteins still interacting with the target protein

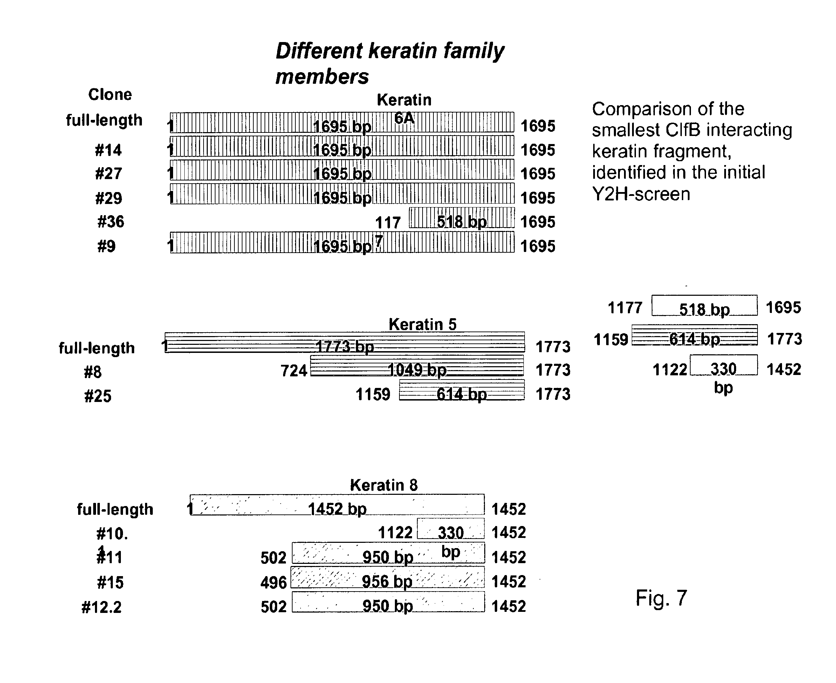

This mini-library exemplifies a specific (because specialized for a distinct target, CIfB) ORFomer-library. The ORFomer library is mutagenized (5-primed deletions). However, the ORFomer library has not undergone iterative rounds of repeated mutagenesis and selections. All constituents of this mini-library are tested directly in a binary manner against CIfB.
- The identified interactions between CIfB and KRT6A, KRT8, and KRT5 (cf Table 1) were used for the experiment.
- The interacting KRT-genes were compared in order to identify the plasmid harbouring the shortest KRT gene-fragment from the initial Y2H-screen. See Figure 7
- KRT8 was identified as the smallest DNA-fragment isolated from the initial Y2H-screen. The clone with the smallest CIfB interacting DNA-insert is called clone cIb10.1
- Clone clb10.1 was used as template for primer-design. PCR-primers were designed to lead to N-terminal truncations of the resulting protein. The cIb10.1 DNA-template contains a 330 base pair DNA-fragment of the gene coding region of KRT8 (corresponds to a peptide of 110 amino acids).
- The first primer-pair binds 45 bp downstream of the cIb10.1 DNA-template resulting in a 45 bp shortened DNA-fragment after PCR (the generated PCR-product contains a gene coding sequence of 285 bp). The resulting peptide encoded from this DNA is 95 amino acids. The PCR-product was cloned into an empty Y2H-prey plasmid, pGADT7. The plasmid was sequenced and the clone was named Frag1.
- The second primer pair amplifies a PCR-product, which contains a gene coding sequence of 144 bp, which corresponds to a peptide of 48 amino acids. The PCR-product was cloned into an empty Y2H-prey plasmid, pGADT7. The plasmid was sequenced and the clone was named Frag2.
- The third primer pair still amplifies the same gene coding sequence of KRT8, however the 3-primed untranslated region was removed. The 3-primed untranslated region is 241 bp long and not relevant for the interaction with CIfB protein. The removal of this 241 region was achieved by using a PCR-reverse primer, which binds upstream of this region in the DNA-template of clone 10.1. The resulting peptide, when translated from this PCR-product is still 48 amino acids. The PCR-product was cloned into an empty Y2H-prey plasmid, pGADT7. The plasmid was sequenced and the clone was named Frag2-CDS.
- The fourth primer pair amplifies a PCR-product of 84 bp, which when translated into a protein will correspond to a peptide of 28 amino acids. The PCR-product was cloned into an empty Y2H-prey plasmid, pGADT7. The plasmid was sequenced and the clone was named Frag2-binding domain.
- The fifth primer pair amplifies a PCR-product of 78 bp; when translated into a protein this fragment will correspond to a peptide of 26 amino acids. The PCR-product was cloned into an empty Y2H-prey plasmid, pGADT7. The plasmid was sequenced and the clone was named Frag3.
- After PCR, the fragments were separated by gel-electrophoresis and the DNA was extracted from the gels by a commercial gel-extraction kit. The DNA was cut by restriction enzymes and cloned into the Y2H-plasmid pGADT7. The cloning reaction was introduced by plasmid transformation into ***E.coli.*** The transformation mixture was plated out onto plasmid-selective media containing the appropriate antibiotic. Growing colonies were inoculated into liquid media containing the same antibiotic, and the ***E.coli*** liquid culture was plasmid-purified and sequenced for confirmation.
- Pure plasmid DNA from all constructs (Frag1, Frag2, Frag2-CDS, Frag2-binding domain, Frag3, cIb10.1 clone, as well as full-length KRT6A, full-length KRT8, and full-length KRT5) were mixed to obtain a mini-library of different shortened fragments from the same interactor of CIfB. Equal molar ratios were mixed per vial.
- In order to identify the smallest DNA-fragment still able to interact with CIfB when translated into a peptide, a systematic Y2H analysis was performed with all constructs directly in binary combinations. The results showed that the shortest fragment encoding a peptide still binding to ClfB encodes a peptide of 28 amino acids. See Figure 3
- All shortened constructs except of the last construct Frag3 (encoding a peptide of 26 amino acids) were still able to interact with CIfB. See Table 6 for detailed information on systematic mutagenesis and the exact nucleic acid sequence of resulting constructs. Table 7 shows all peptide sequences, which are translated from the plasmid constructs and tested for interaction with CIfB protein.

**Table 6: Exact nucleic acid sequences inserted in a plasmid for translation into a peptide, generated by systematic mutagenesis. Start and Stop codon of the KRT8 ORF is indicated in italic, untranslated region of the gene is indicated in bold, DNA coding for a peptide is underlined**

| **Construct** | **Nucleic acid sequence** |
|---|---|
| KRT 8 gene Full-length ORF | ATGTCCATCAGGGTGACCCAGAAGTCCTACAAGGTGTCCACCTCTGGCCCCGGGC CTTCAGCAGCCGCTCCTACACGAGTGGGCCCGGTTCCCGCATCAGCTCCTCGAGCT TCTCCCGAGTGGGCAGCAGCAACTTTCGCGGTGGCCTGGGCGGCTATGGTGG GGCCAGCGGCATGGGAGGCATCACCGCAGTTACGGTCAACCAGAGCCTGCTGAGC CCCCTTGTCCTGGAGGTGGACCCCAACATCCAGGCCGTGCGCACCCAGGAGAAGG AGCAGATCAAGACCCTCAACAACAAGTTTGCCTCCTTCATAGACAAGGTACGGTTCC TGGAGCAGCAGAACAAGATGCTGGAGACCAAGTGGAGCCTCCTGCAGCAGCAGAA GACGGCTCGAAGCAACATGGACAACATGTTCGAGAGCTACATCAACAACCTTAGGC GGCAGCTGGAGACTCTGGGCCAGGAGAAGCTGAAGCTGGAGGCGGAGCTTGGCAA |
| | CATGCAGGGGCTGGTGGAGGACTTCAAGAACAAGTATGAGGATGAGATCAATAAGC GTACAGAGATGGAGAACGAATTTGTCCTCATCAAGAAGGATGTGGATGAAGCTTACA TGAACAAGGTAGAGCTGGAGTCTCGCCTGGAAGGGCTGACCGACGAGATCAACTTC CTCAGGCAGCTATATGAAGAGGAGATCCGGGAGCTGCAGTCCCAGATCTCGGACAC ATCTGTGGTGCTGTCCATGGACAACAGCCGCTCCCTGGACATGGACAGCATCATTG CTGAGGTCAAGGCACAGTACGAGGATATTGCCAACCGCAGCCGGGCTGAGGCTGA GAGCATGTACCAGATCAAGTATGAGGAGCTGCAGAGCCTGGCTGGGAAGCACGGG GATGACCTGCGGCGCACAAAGACTGAGATCTCTGAGATGAACCGGAACATCAGCCG GCTCCAGGCTGAGATTGAGGGCCTCAAAGGCCAGAGGGCTTCCCTGGAGGCCGCC ATTGCAGATGCCGAGCAGCGTGGAGAGCTGGCCATTAAGGATGCCAACGCCAAGTT GTCCGAGCTGGAGGCCGCCCTGCAGCGGGCCAAGCAGGACATGGCGCGGCAGCT GCGTGAGTACCAGGAGCTGATGAACGTCAAGCTGGCCCTGGACATCGAGATCGCCA CCTACAGGAAGCTGCTGGAGGGCGAGGAGAGCCGGCTGGAGTCTGGGATGCAGAA CATGAGTATTCATACGAAGACCACCAGCGGCTATGCAGGTGGTCTGAGCTCGGCCT ATGGGGGCCTCACAAGCCCCGGCCTCAGCTACAGCCTGGGCTCCAGCTTTGGCTCT GGCGCGGGCTCCAGCTCCTTCAGCCGCACCAGCTCCTCCAGGGCCGTGGTTGTGA AGAAGATCGAGACACGTGATGGGAAGCTGGTGTCTGAGTCCTCTGACGTCCTGCCC AAGTGAACAGCTGCGGCAGCCCCTCCCAGCCTACCCCTCCTGCGCTGCCCCAGAG CCTGGGAAGGAGGCCGCTATGCAGGGTAGCACTGGGAACAGGAGACCCACCTGA GGCTCAGCCCTAGCCCTCAGCCCACCTGGGGAGTTTACTACCTGGGGACCCCCCT TGCCCATGCCTCCAGCTACAAAACAATTCAATTGCTTTTTTTTTTGGTCCAAAATA AAACCTCAGCTAGCTCTGCCAAACCC |
| Clone 10.1 | CAGGAGCTGATGAACGTCAAGCTGGCCCTGGACATCGAGATCGCCACCTACAGGAA GCTGCTGGAGGGCGAGGAGAGCCGGCTGGAGTCTGGGATGCAGAACATGAGTATT CATACGAAGACCACCAGCGGCTATGCAGGTGGTCTGAGCTCGGCCTATGGGGGCCT CACAAGCCCCGGCCTCAGCTACAGCCTGGGCTCCAGCTTTGGCTCTGGCGCGGGC TCCAGCTCCTTCAGCCGCACCAGCTCCTCCAGGGCCGTGGTTGTGAAGAAGATCGA GACACGTGATGGGAAGCTGGTGTCTGAGTCCTCTGACGTCCTGCCCAAGTGAACAG CTGCGGCAGCCCCTCCCAGCCTACCCCTCCTGCGCTGCCCCAGAGCCTGGGAAG GAGGCCGCTATGCAGGGTAGCACTGGGAACAGGAGACCCACCTGAGGCTCAGCC CTAGCCCTCAGCCCACCTGGGGAGTTTACTACCTGGGGACCCCCCTTGCCCATGC CTCCAGCTACAAAACAATTCAATTGCTTTTTTTTTTTGGTCCAAAATAAAACCTCAG CTAGCTCTGCCAAACCC |
| Frag 1 | ACCTACAGGAAGCTGCTGGAGGGCGAGGAGAGCCGGCTGGAGTCTGGGATGCAGA ACATGAGTATTCATACGAAGACCACCAGCGGCTATGCAGGTGGTCTGAGCTCGGCC TATGGGGGCCTCACAAGCCCCGGCCTCAGCTACAGCCTGGGCTCCAGCTTTGGCTC TGGCGCGGGCTCCAGCTCCTTCAGCCGCACCAGCTCCTCCAGGGCCGTGGTTGTG AAGAAGATCGAGACACGTGATGGGAAGCTGGTGTCTGAGTCCTCTGACGTCCTGCC CAAGTGAACAGCTGCGGCAGCCCCTCCCAGCCTACCCCTCCTGCGCTGCCCCAGA |
| | GCCTGGGAAGGAGGCCGCTATGCAGGGTAGCACTGGGAACAGGAGACCCACCTGA GGCTCAGCCCTAGCCCTCAGCCCACCTGGGGAGTTTACTACCTGGGGACCCCCCTT GCCCATGCCTCCAGCTACAAAACAATTCAATTGCTTTTTTTTTTGGTCCAAAATAAAA CCTCAGCTAGCTCTGCCAAACCC |
| Frag 2 | TACAGCCTGGGCTCCAGCTTTGGCTCTGGCGCGGGCTCCAGCTCCTTCAGCCGCAC CAGCTCCTCCAGGGCCGTGGTTGTGAAGAAGATCGAGACACGTGATGGGAAGCTGG TGTCTGAGTCCTCTGACGTCCTGCCCAAGTGAACAGCTGCGGCAGCCCCTCCCAGC CTACCCCTCCTGCGCTGCCCCAGAGCCTGGGAAGGAGGCCGCTATGCAGGGTAG CACTGGGAACAGGAGACCCACCTGAGGCTCAGCCCTAGCCCTCAGCCCACCTGG GGAGTTTACTACCTGGGGACCCCCCTTGCCCATGCCTCCAGCTACAAAACAATTCA ATTGCTTTTTTTTTTGGTCCAAAATAAAACCTCAGCTAGCTCTGCCAAACCC |
| Frag 2-CDS | TACAGCCTGGGCTCCAGCTTTGGCTCTGGCGCGGGCTCCAGCTCCTTCAGCCGCAC CAGCTCCTCCAGGGCCGTGGTTGTGAAGAAGATCGAGACACGTGATGGGAAGCTGG TGTCTGAGTCCTCTGACGTCCTGCCCAAGTGA |
| Frag 2 binding domain | TACAGCCTGGGCTCCAGCTTTGGCTCTGGCGCGGGCTCCAGCTCCTTCAGCCGCAC CAGCTCCTCCAGGGCCGTGGTTGTGAAGA |
| Frag 3 | CAGGGCCGTGGTTGTGAAGAAGATCGAGACACGTGATGGGAAGCTGGTGTCTGAGT CCTCTGACGTCCTGCCCAAGTGA |

**Table 7: Sequences of peptides encoded by the constructs used in experiments of example 4**

| Construct | Resulting peptide | Length of peptide |
|---|---|---|
| Clone 10.1 | QELMNVKLALDIEIATYRKLLEGEESRLESGMQNMSIHTKTTSGYAGG LSSAYGGLTSPGLSYSLGSSFGSGAGSSSFSRTSSSRAVVVK- KIETRDGKLVSESSDVLPK | 110 amino acids |
| Frag 1 | TYRKLLEGEESRLESGMQNMSIHTKTTSGYAGGLSSAYGGLTSPGLS YSLGSSFGSGAGSSSFSRTSSSRAVVVKKIETRDGKLVSESSDVLPK | 95 amino acids |
| Frag 2 | YSLGSSFGSGAGSSSFSRTSSSRAVVVKKIETRDGKLVSESSDVLPK | 48 amino acids |
| Frag 2-CDS | YSLGSSFGSGAGSSSFSRTSSSRAVVVKKIETRDGKLVSESSDVLPK | 48 amino acids |
| Frag 2 binding domain | YSLGSSFGSGAGSSSFSRTSSSRAVVVK | 28 amino acids |
| Frag 3 | RAVVVKKIETRDGKLVSESSDVLPK | 26 amino acids |

### Example 5: Production of specific and selected peptides (mature ORFomers) by iterative rounds of mutagenesis and selection

Mutagenesis followed by selection and further rounds of mutagenesis & selection of minimal interacting peptides in an ORFomer library results in specific and selected ORFomer, selected for binding to a specific target.

### 5.1. Exemplified by systematic mutagenesis:

Shortening of the gene encoding the interacting protein, resulting in N- and C-terminal truncations, until the interaction is lost to identify the smallest peptide derived from the binding proteins still interacting with the target protein

The mini-library constructed in example 4 was mutagenized, however has not undergone iterative rounds of selection & mutagenesis. To demonstrate, that repeated rounds of selection can eliminate non-binders from binders 2 rounds of selection within the Y2H-system were performed.
- The mini-library was transformed together with the plasmid containing the target-protein encoding CIfB into a Y2H-strain (AH109), and the transformation was plated onto yeast-media plates selecting PPI
- After 5 days of incubation all growing colonies were scraped up from the plates and plasmids encoding for the interactors were isolated.
- The isolated plasmids were transformed into ***E.coli*** for amplification, and plasmid purification was done by commercial plasmid purification kits.
- The plasmid mixture was re-introduced into a Y2H-strain (AH109) and again plated onto yeast-media plates selecting PPI. After 5 days of incubation, growing colonies were scraped up from the plates and plasmids encoding for the interactors were isolated from yeast.
- The isolated plasmids were again transformed into ***E.coli*,** and purified by commercial plasmid purification kits.
- An aliquot of this plasmid mixture was transformed into ***E.coli*,** and plated out onto bacterial media containing the appropriate antibiotic for selection of plasmids encoding the interactors of ClfB).
- In total, 100 plasmids from 100 different ***E.coli*** colonies were isolated, and the DNA-inserts within the plasmids were sequenced.

The plasmid harbouring the sequence encoding the shortest KRT fragment still capable of interaction was Frag2-binding domain encoding a peptide of 28 amino acids. This clone was identified 18 times within the 100 different clones and Frag2-CDS was identified 28 times within the 100 plasmids. No single clone of the 26 amino-acid peptide encoding plasmid (Frag3) was identified. This experiment demonstrates that two rounds of selection within the Y2H-system are sufficient to eliminate non-binders from a binder population within a functional (here for CIfB) ORFomer library (see Table 8)

**Table 8: Elimination of non-binders by selection**

| **Construct** | **Identified X-times by sequencing** | **Interaction capable with ClfB in the Y2H-system** |
|---|---|---|
| Clone 10.1 | 8 | Yes |
| Frag 1 | 12 | Yes |
| Frag 2 | 25 | Yes |
| Frag 2-CDS | 28 | Yes |
| Frag 3 | 0 | No |
| Frag 2-binding domain | 18 | Yes |
| Sequencing failed | 9 | |
| Total | 100 clones | |

### 5.2. Exemplified by random mutagenesis

A Y2H-compatible 'restriction-fragment gene-library' was produced from cDNA encoding the interacting human proteins, with the aim to identify (using the Y2H system) the smallest peptide derived from the binding proteins still interacting with the target protein. Chemical synthesis is used to confirm the nature of the binding peptide. The peptide can be used for a variety of applications.

Here, both iterative rounds of selection and mutagenesis from a specific but unselected (still containing non-binders and weak binders) ORFomer library were performed. The resulting peptide is a selected mature ORFomer.

### 5.2.1. Mutagenesis

- From the FnbB interacting proteins, which were identified in the initial Y2H-screen (cf. Table1), six candidates were chosen.
- All candidates are proteins that have been confirmed by immuneprecipitation to be binders (see Section 5.2)
- From these six different candidates, the ORFs have been cloned into Y2H-plasmid pGADT7 (the genes HSPC118, CCT-beta, sialidase Neu1, PNAS-110, beta-actin and FLNA)
- Each of the plasmids were used as template for a PCR reaction with the primers Fw: 5'-CTATTCGATGATGAAGATACCCCA - 3', Rw: 5'- GTGAACTTGCGGGGTTTTTCAG - 3'
- The PCR products were purified by agarose gel electrophoresis and a commercial DNA and Gel Band Purification Kit
- The PCR products were mixed in an equimolar ratio. The final mixture contained 3 µg of DNA
- The DNA-mixture was digested by the following highly frequent cutting restriction enzymes: Mspl, Acil, Maell, HinPI. These enzymes recognize a specific sequence of four bases. A partial digest was performed in order to contain overlapping restriction fragments and to not over-digest the DNA into useless fragments of 50 or less deoxynucleotides.
- All numerated enzyme cutting sites are compatible with the restriction site of the restriction enzyme ClaI, which is used for the vector preparation.
- Full and partial digests of the PCR-products and purified DNA of 50 bp - 200 bp fragment length were pooled by agarose gel electrophoresis and the use of DNA Purification Kits
- The DNA-fragments were cloned by T4-ligase ligation into the Clai-restriction enzyme pre-cut Y2H-plasmid pGADT7.
- Four ligation reactions were performed
- Each of the legation reaction was transformed into chemically competent E. coli cells and plated out onto plasmid-selective media containing the appropriate antibiotics.
- Arising colonies were scraped from the Petri-dishes and pooled. The E. coli pool was plasmid purified by a commercial plasmid preparation kit.
- The number of growing colonies per transformation is shown in Table 9
- The mini-library (called mini-library I) contains a complexity of ~ 100.000 independent colonies each harbouring a distinct DNA-fragment from any of the six candidate interactors.
- The cloning efficiency was >90%

**Table 9: Complexity of the mini-library I shown by numbers of growing E.coli colonies**

| **Ligation** | **obtained total amounts of E. coli transformants on Petridishes** |
|---|---|
| 1 | 17.000 |
| 2 | 21.000 |
| 2 | 26.000 |
| 3 | 36.000 |
| In sum | ~100.000 independent E. coli colonies obtained |

### 5.2.2. Iterative rounds of selection

- The mini-library was transformed together with the target gene harbouring plasmid (FnbB) into the Y2H-strain (AH109)
- Yeast transformants were plated out onto dishes containing PPI-selective yeast media
- Growing yeast colonies were scraped up and pooled
- Plasmid from the pooled yeast cells was isolated and transformed for propagation into E.coli, and plasmid-DNA was isolated
- Isolated plasmid was re-transformed into the Y2H-strain (AH109)
- Yeast transformants were plated out onto PPI-selective yeast-media
- Growing yeast colonies were scraped up and pooled
- Plasmid from the pooled yeast cells was isolated and transformed for propagation into E.coli, and plasmid-DNA was isolated
- The above procedure represents 2 rounds of selection within the Y2H-system
- The plasmid DNA, which should be free from sequences encoding non-binfing ORFomers, was used for an additional round of mutagenesis (again by the restriction enzyme-based strategy described in 8.2.1.). However, the mini-library II produced, was less complex than the first one and contained only 10.000 individual clones.
- The library was subject to one round of selection within the Y2H-system.
- Resulting yeast colonies, were plasmid isolated, transformed into E.coli and sequenced for identification of the DNA encoding an interacting minimal peptide for FnbB
- The number of selection rounds and mutagenesis rounds can be varied. Selection under more or less stringent conditions can be used (e.g. using compounds such as 3-amino-triazole), more or fewer rounds of selection, and the type of mutagenesis can be done differently each time and can be adjusted to the targets (e.g. if target is a sticky protein, which needs stronger conditions, or a high number of targets are used per experiment, etc.)
- Table 10 shows the sequence of the interacting minimal peptides, the mature ORFomers

**Table 10: FnbB interacting peptides encoded by DNA fragments contained in the Y2H plasmids**

| **Nucleic acid sequence** | **Peptide resulting from the nucleic acid sequence** | **Number of Amino acids** | **Fragment of gene X** |
|---|---|---|---|
| CGTAGAGCTGGGGGGCC TGCTCCTCTCCATCCATG CTGCCCTCCAGGGTTGC CAGGGAT- GAATAGCCACTGGGGCC TGGCCATAGCTGGACTGT CTCTTTCCGATACG | RRAGG- PAPLHPCCPPGLPGMNSHW GLAIAGLSLS | 34 | Sialidase NEU1 |
| CGGAAAGAGACAGTCCA GCTATGGCCAGGCCCCA GTGGCTATTCATCCCTGG CAACCCTGGAGGGCAG- CATGGATGGAGAGGAG- CAGGCCCCCCAGCTCTA CG | RKETVQLWPGPSGYSS- LATLEGSMDGEEQAPQLY | 34 | Sialidase NEU1 |
| CGCGTCACCTA- TACCCCCATGGCACCTG GCAGCTACCTCATCTCCA TCAAGTACGGCG | RVTYTPMAPGSYLISIKYG | 19 | FLNA |

### Example 6: Binding specificity of the peptides demonstrated by using two different technologies: the molecular genetic Y2H test system and the biochemical pull-down analysis

### 6.1. Specificity of the peptides in the Y2H-system

- The plasmids encoding the peptides and the proteins were transformed into the Y2H-system reporter strain AH109. The transformation was always a combination of two plasmids.
- The transformation reaction was plated out onto yeast media selective for both plasmids, but not selective for the PPI.
- After 5 days of incubation, growing colonies were mixed. Five colonies from each plate were randomly taken and the colonies were resuspended in sterile water.
- From this mixture, an aliquot was transferred onto yeast media, restrictive for PPI. The transferred volume, was dropped onto a yeast plate and after the fluid soaked completely into the solid media, the plates were incubated for 4-days at 28°C

Following yeast transformations were performed:
a) The DNA encoding the smallest ClfB interacting peptide, which is located within the Y2H-plasmid pGADT7, called Frag2-binding domain, is transformed together with the plasmid harbouring the DNA for ClfB protein into the Y2H-reporter strain (AH109).
b) The DNA encoding the smallest ClfB interacting peptide, which is located within the Y2H-plasmid pGADT7, called Frag2-binding domain, is transformed together with the plasmid harbouring only DNA for the binding domain of yeast protein GAL4 into the Y2H-reporter strain (AH109).
c) The DNA coding for the peptide in the Frag2-binding domain plasmid is isolated by PCR and cloned into the Y2H-plasmid pGBKT7. This plasmid is transformed together with the smallest CIfB interacting peptide-encoding DNA, which is located within the Y2H-plasmid pGADT7, called Frag2-binding domain into Y2H-reporter strain (AH109).
d) The DNA coding for the peptide in the Frag2-binding domain plasmid is isolated by PCR and cloned into the Y2H-plasmid pGBKT7. This plasmid is transformed together with the plasmid harbouring only DNA for the activation domain of yeast protein GAL4 into the Y2H-reporter strain (AH109).
e) The plasmid containing the DNA coding for CtfB is transformed together with the plasmid harbouring only DNA for the activation domain of yeast protein GAL4 into the Y2H-reporter strain (AH109).

Figure 4 shows the results of binding as tested by the Y2H-system, and gives an overview of the tested peptides and proteins. The peptide of 28 amino acids produced in yeast from the Frag2-binding domain DNA does only interact with ClfB protein produced in yeast. This peptide neither interacts with the activation domain of GAL4 protein encoded in the Y2H-plasmid nor with the binding-domain of GAL4 protein encoded in the Y2H-plasmid. The 28 amino acid-peptide does not interact with itself, thus it is neither a homodimerizing peptide nor a sticky protein. The results clearly demonstrate, that the peptide binds specifically only to its target, ClfB, when expressed in yeast.

### 6.2. Specificity of the peptides in the in vitro-pull down analysis

### 6.2.1. Recombinant protein production

- The DNA-sequence coding for ClfB was cloned into a plasmid PET160 (a plasmid suitable for recombinant protein expression in E.coli and HIS-tag purification). The plasmid harbours a so-called HIS-tag, which is a DNA sequence coding for a stretch of six histidine residues. Each DNA cloned in frame with the His-tag into PET160 is translated in E.coli to a recombinant fusion protein containing six histidine residues and can be purified by affinity materials coated with Ni2+ ions, The His-tag will be C-terminally located in the recombinant protein.
- The DNA-sequence located in the plasmid pGADT7 coding for a peptide (Frag2-CDS, a peptide of 48 amino acids) that can interact with ClfB in the Y2H-system, was isolated by PCR with specific primers and cloned into the plasmid pDEST15. This plasmid is suitable for recombinant protein expression in E.coli and GST-tag purification. The plasmid harbours a so-called GST-tag, which is a DNA sequence coding for glutathione S-transferase, a 34 kd protein having increased affinity to glutathione. Each DNA cloned in frame with the GST-tag in pDEST15 is translated in E.coli to a recombinant fusion protein containing N-terminally fused the glutathione S-transferase.
- Both plasmids were transformed for protein production into E.coli strain BL21 and induced with IPTG for high level protein expression.
- Additionally, recombinant purified GST-protein was purchased from a company
- From the recombinant proteins produced in 50 ml E.coli liquid culture 1 ml was used for pull-down analysis.

### 6.2.2. Pull-down experiment demonstrating specificity of the peptide

- The pull-down was performed via the His-tag of the His-tagged recombinant proteins. This means His-tagged proteins are immobilized on magnetic beads coated with Ni-ions and purified GST-taged proteins/peptides are allowed to bind to the pre-bound His-tag protein/peptide. After several washing steps, antibodies specific for the GST-tag are used for detection of the GST-tagged binder.
- 1 ml of an IPTG-induced E.coli liquid culture expressing the recombinant His-tagged ClfB is lysed and incubated with Ni-ion bound magnetic particles.
- Unbound regions are blocked with BSA
- Purified recombinant GST-taged peptide is added (purified from E.coli BL21, containing pDEST15 which harbours the DNA for the ClfB binding peptide, Frag2-CDS). The GST-tagged peptide was purified by using a commercial GST-tag purification kit.
- After several washing steps the remaining proteins are eluted from the magnetic beads
- The eluted protein/peptide mixture is loaded on a SDS-page gel, separated, transferred onto a membrane, and probed with GST-tag specific antibodies and bound antibodies are detected by chemiluminescence signals on chemiluminescence films.
- The following combinations of recombinant proteins/peptides were used in the pull-downs:
   a) BSA+GST-tagged 48-aminoacid long peptide
   b) His-tagged ClfB + GST-tagged 48-amino acid-peptide
   c) Purified GST+GST-tagged 48-amino acid-peptide

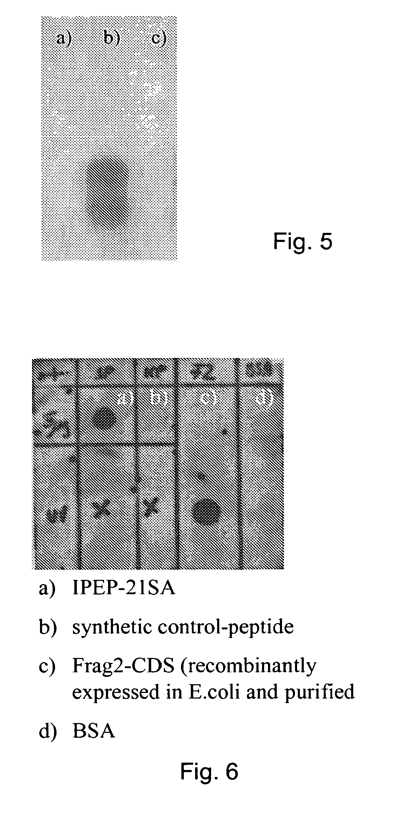

The results show that the GST-tagged peptide of 48-amino acids is only pulled down (co-purified) with the His-tagged ClfB protein. Neither BSA coated- nor GST coated magnetic beads could pull down the GST-tagged 48-aminoacid peptide. The results clearly demonstrate that the recombinant peptide of 48-amino acids binds specifically to its target the ClfB protein recombinantly produced in ***E. coli*** (see figure 5).

### Example 7: Inhibition of addressed PPI by peptides (PPI-inhibitory peptides)

### 7.1. Specific binding of the PPI-inhibitory peptide to the target ClfB demonstrated by dotblot far-western approach.

- By using Y2H-analysis results of ClfB interacting and non-interacting peptides and comparing their amino acid-sequences, we extracted a peptide of 21 amino acids (cf. Table 11), which is the core element interacting with ClfB protein. The sequence of this peptide, called IPEP-21 SA is N-SYSLGSSFGSGAGSSSFSRTS.
- The peptide was made also by chemical synthesis
- Additionally, we designed a control-peptide, which is located in a region of KRT8 that does not interact with ClfB in the Y2H-system. This peptide was also synthesized. The sequence of the control peptide is N-EQRGELAIKDANAKLSELEAAL
- The synthetic peptides were dissolved in sterile distilled water and 5 µg of the synthetic peptides were spotted onto a membrane together with the recombinant GST-tagged peptide of 48-amino acids produced by plasmid construct Frag2-CDS. and BSA was used as negative control.
- The membrane was blocked and incubated with recombinant His-tagged ClfB protein
- After binding of ClfB protein to interacting peptides, the blot was washed and incubated with antibody specific for the His-tag of ClfB.
- After washing the membrane, a secondary antibody was added, which is conjugated with alkaline phosphatase, and a signal was detected by chemiluminescence with the appropriate substrate and film development.
- Figure 6 shows that recombinant ClfB could bind to the synthetic peptide IPEP21-SA as well as to the recombinant 48-amino acid-peptide (Frag2-CDS). No binding was detected for BSA and no binding was detected for the synthetic control peptide. The results again demonstrate that the binding is specific.

Peptides were spotted onto a protein binding membrane and incubated with recombinant His-tagged ClfB protein. The irreversibly bound peptides can be targeted by additionally added ClfB. Bound ClfB protein is detected by a His-tag specific antibody (see fig. 7).

### 7.2. PPI-inhibition by the inhibitory peptide

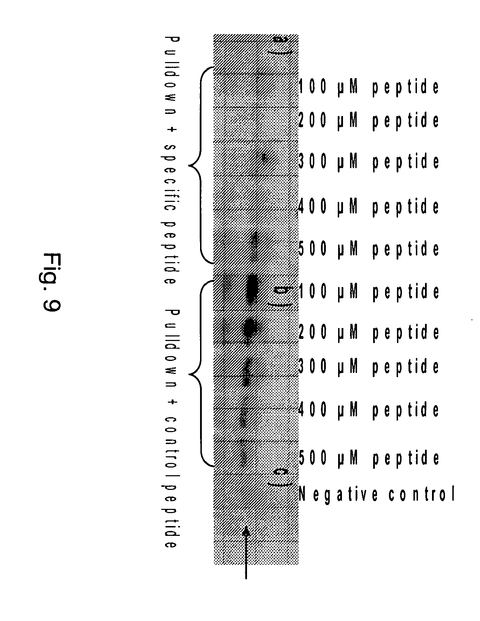

- To show that the peptides developed can interrupt the specific PPI between KRT8 and ClfB an in vitro pulldown-competition test was performed.
- Recombinant His-taged ClfB protein was immobilized on magnetic beads (specific for His-tag binding) and purified recombinant GST-tagged 48-amino acid-keratin fragment (Frag2-CDS) was added with IPEP-21SA or with the control peptide.
- After several washing steps, the bound protein fraction was eluted and subjected to Western blot analysis with an antibody specific for GST-tagged proteins.
- The results show, that recombinant ClfB pulls down more GST-tagged keratin fragment in the presence of the control peptide and in the absence of any peptide, than in the presence of 1µM of IPEP-21SA. Thus, IPEP-21SA competes with the GST-tagged keratin-derived peptide of 48-amino acid for binding to ClfB and therefore displaces or/and blocks binding. The concentration of 1 µM IPEP-21SA inhibits competitively the binding of GST-tagged keratin fragment to ClfB-protein by ~40%. Relative changes in pulled-down GST-tagged keratin fragment is quantified by densitometry using a flatbed scanner and the ImageJ software, provided by Wayne Rasband, National Institutes of Health. (ImageJ: http://rsb.info.nih.gov/ij/) See figs. 8 and 9

### Example 8:

- All genes coding for the used recombinant proteins were cloned into the plasmid pDEST15. This plasmid is suitable for recombinant protein expression in ***E.coli*** and GST-tag purification. The plasmid harbours a so-called GST-tag, which is a DNA sequence coding for glutathione S-transferase, a 34 kd protein having increased affinity to glutathione. Each DNA cloned in frame with the GST-tag in pDEST15 is translated in ***E.coli*** to a recombinant fusion protein containing N-terminally fused the glutathione S-transferase. Both plasmids were transformed for protein production into ***E.coli*** strain BL21 and induced with IPTG for high level protein expression.
- The GST tagged peptide was purified by using a commercial GST-tag purification kit from Promega company
- All synthetic peptides were chemically synthesized and obtained from a commercial supplier
- Human elastin protein and fibrinogen protein were commercially obtained
- Proteins and peptides used:
   - Bovine serum albumine (BSA) (500ng/µl). This protein served as negative control 1
   - Affinity purified, recombinantly in *E. coli* produced GST-tagged Frag2-CDS (49ng/µl) the amino acid sequence of Frag2-CDS is N-YSLGSSFGSGAGSSSFSRTSSSRAVVVKKIETRDGKLVSESSDVLPK-C
   - Affinity purified, recombinantly in *E. coli* produced GST-tagged S. *aureus* protein, methionine sulfoxide reductase msrB, NCBI protein GI:54041496, was diluted to a final concentration of 30 ng/µl. This protein served as negative control 2
   - Affinity purified, recombinantly in *E. coli* produced GST-tagged *S*. *aureus protein,* NADPH-dependent 7-cyano-7-deazaguanine reductase, NCBI protein GI:81781951, was diluted to a final concentration of 61 ng/µl. This protein served as negative control 3
   - Human aortic elastin protein, 1 mg elastin was purchased from the company Elastin Products company, product number HA587 and solubilized in 1 ml H₂O to get the final concentration of 1 µg/µl. This protein served as positive control 1 because elastin protein is known to bind to *S*. *aureus.*
   - Human fibrinogen protein was purchased from the company Enzyme Research Laboratories, product number FIB 1 3120L; 1g fibrinogen was solubilized in 50 ml H₂O to get the final concentration of 2 µg/µl. This protein served as positive control 2 because fibrinogen protein is known to bind to *S*. *aureus*
   - Synthetic IPEP-21SA (4,2µg/µl), the sequence of IPEP-21SA is is N-SYSLGSSFGSGAGSSSFSRTS
   - Synthetic control peptide (4,7µg/µl), the sequence of the control peptide is N-EQRGELAIKDANAKLSELEAAL
- Immobilizer™ Amino 96 well plates (Modules Clear) from the commercial supplier Nunc (product number 436006) were coated with proteins and peptides, which were diluted in 0,02% sodium carbonat buffer (pH 9,6) to get a final concentration of 5 ng/µl. 200 µl of protein and peptide solutions were used for coating, which corresponds to 1 µg total amount of protein or peptide.
- coating was done over night at 4°C, then the supernatant was removed and each well was blocked with 2mg/ml BSA, 2h at 37°C and washed 3 times with 200 µl phosphate buffered saline (PBS)
- in the meantime, an ***S*. *aureus*** culture was grown to a density of 0,5 OD₆₀₀
   - corresponding to 230 000 cells/µl)
- 300 µl of the ***S. aureus*** culture was added to each well and incubated 2 hours at 37°C
- after incubation unbound ***S. aureus*** cells were removed and the wells were washed 4 times with 200µl PBS
- ***S**. **aureus*** cells, which were bound to the coated wells were fixed with 25 % formaldehyde solution, for 5 minutes at room temperature
- After removal of the formaldehyde solution, wells were dried for 5 minutes at room temperature
- for detection of bound ***S. aureus*** cells, 200µl crystal violet (0,5%) solution was added to each well and incubated for 30 minutes at room temperature
- wells were washed with 200µl H₂O
- dye was dissolved with 10 % acetic acid by shaking 30 minutes at RT
- absorbance was measured at 595 nm

The results show, that recombinant GST-tagged keratin fragment (recombinant ORFomer) binds strongly to living pathogen cells with affinities comparable to published virulence factor substrates as shown by an adhesion assay. The results also show, that IPEP-21SA (the synthetic ORFomer) binds strongly to living pathogen cells compared to synthetic control peptide.

### Example 9: The method according to the present invention was performed on the peptide YSLGSSFGSGAGSSSFSRTSSSRAVWK, the original binding domain to ClfB, in order to provide molecules with increased relative protein interaction strength.

### 1. Construction of a mutagenesis-peptide library

1.1 The nucleic acid sequence which encodes for the original binder peptide (amino acid sequence: YSLGSSFGSGAGSSSFSRTSSSRAVVVK) was amplified from the Y2H prey plasmid pGADT7 by PCR.
1.2 The PCR product was mutagenized by error-prone PCR with the commercially available product Diversify^{®} PCR Random Mutagenesis Kit from Clontech.
1.3 The mutagenized PCR product was cloned into the Y2H prey vector pGADT7 by restriction enzyme based cloning. At least 8x10⁶ mutagenized clones were generated.
1.4. The mutagenesis procedure was repeated to gain a highly diverse peptide library. Random E. coli clones which harbour a mutagenized peptide encoding nucleic acid sequence were analysed by plasmid isolation and subsequent sequencing. Equal distribution of mutations can be seen over the complete length of the peptide (Table 11).

### 2. Selection in the Y2H system

2.1. The mutagenesis library was introduced into the Y2H strain AH 109 by lithium acetate based transformation and a total number of 8,6x10⁶ individual yeast transformants were generated. Yeast trans-formantions were plated out onto protein-interaction selective media (yeast selective media lacking tryptophan, leucine, Adenine, histidine and supplemented with 50 mM 3AT). Colonies that appeared on the plates were harvested from the plates by scraping and pooled to yield a mixture of pre-selected differently mutagenized original binders expressed in AH109 yeast cells (7x10⁶ cells/µl).
2.2 Two alliquots of the pooled colonies were taken (corresponding to 7x10⁷ cells) and inocculated in 2 different liquid Y2H selection media (25 ml of yeast selective media lacking tryptophan, leucine, histidine, adenine supplemented with 250 mM 3AT and additionally 25 ml of yeast selective media lacking tryptophan, leucine, histidine, adenine and 3AT).
2.3 The yeast liquid cultures were incubated 1 week at 28°C and reached a cell density of (~10¹¹ cells in 25 ml in 0 mM 3AT and 5x10⁹ in 250 mM 3AT).
2.4. Steps 2.2 and 2.3 were repeated (again an alliquot of the selected cells was inocculated into the same selection media and grown for 1 week at 28°C).
2.5 Plasmids were isolated from the yeast cell pool and randomly taken clones were sequenced (Tables 12, 13). The observed mutations are not randomly distributed over the complete sequence.
2.6 Additionally, a third alliquot was taken from step 2.1, plated out onto Y2H selective media (yeast selective media lacking tryptophan, leucine, adenine, histidine and containing additionally 50 mM 3AT) and incubated at the very stringent temperature of 37°C (this is a unusual high selection temperature compared to the standard Y2H selection of 28°C).
2.7 After 1 week incubation the plasmids from grown yeast colonies were isolated and sequenced (Table 14).

### 3. Monitoring of the increased relative protein interaction strength in the Y2H system

3.1. The plasmid harbouring the nucleic acid sequence that encodes for the peptide (YSLGSSFGSGAGSSSLGRTSSSRAVVVK) was re-introduced into AH109 cells and tested for the relative interaction strength to ClfB by X-Gal overlay assays and growth strength on 3AT media (Fig. 11). This peptide was called "stronger binder" because it binds with increased affinity to ClfB in the Y2H system compared to the original binder.

### 4. Construction of a mutagenesis-peptide library from selected clones in 2.5

4.1. The same strategy was used to construct a further mutagenesis-peptide library, however, instead of using a single nucleic acid sequence a mixture of nucleic acids was used as template for the error-prone PCR.
4.2. Plasmid isolation from step 2.5 contains a pool of pre-selected strong binders to ClfB, which were used for the error-prone PCR and yielded PCR product was again cloned into the Y2H prey plasmid pGADT7. A total number of 10⁷ mutation clones were obtained.
4.3. This mutagenesis procedure was repeated.

### 5. Selection in the Y2H system

5.1. Steps from 2.1 to 2.5 were repeated twice. Sequenced clones can be seen in table 5.

### 6. Monitoring of the increased relative protein interaction strength in the Y2H system

6.1. The plasmid harbouring the nucleic acid sequence that encodes for the peptide (YSLGSSFGSGAGSSSSSRTSPSRAWVK) was re-introduced into AH109 cells and tested for the relative interaction strength to ClfB by X-Gal overlay assays and growth strength on 3AT media (Fig. 11). This peptide was called "more stronger binder" because it binds with increased strength to ClfB in the Y2H system compared to the original binder.

| Peptide Name | Aminoacid Sequence | Mutagenesis | Selection | Selection | Rounds of Mutagenesis & Selection |
|---|---|---|---|---|---|
| | | | Moderate | Strong | |
| >Frag2binding domain | YSLGSSFGSGAG SSSFSRTSSSRAV VVK | 1 | 1 | 0 | First round |
| | | | | | |
| >F2BD Mutll+ Li g2 8.12.07 | YSLGSSFGS- GAGS[?]SFSR[?]SS SRAVVVK | 1+2 | 1+0 | 0 | Second round |
| >F2BD Mutll+ Li g48.12.07 | YSLGSSFGS- GAGSSSFSRTSS SRAVVVK | 1+2 | 1+0 | 0 | Second round |
| >F2BD Mutll+ Li g58.12.07 | YSLGSSFGS- GAGSSSFSRTSS SRAVVV[?] | 1+2 | 1+0 | 0 | Second round |
| >F2BD Mutll+ Li g6 8.12.07 | YSLGSSFGS- GAGSSSFSRTSS SRAVVVK | 1+2 | 1+0 | 0 | Second round |
| >F2BD Mutll+ Li g7 8.12.07 | YSLGSSFGS- GAGSSSFSRSSS SRAVVVK | 1+2 | 1+0 | 0 | Second round |
| >F2BD Mutll+ Li g8 8.12.07 | YSLGSSF[?]S- GAGSSSFSRTSS [?]RAVVVK | 1+2 | 1+0 | 0 | Second round |
| >F2BD Mutll+ Li g9 8.12.07 | [?]SLGSSFGSGAG SSSFSRTSSSR[?]V VVK | 1+2 | 1+0 | 0 | Second round |
| >F2BD Mutll+ Li g10 8.12.07 | YSLGSSFGS- GAGSS[?]SRTSSS RAVVVK | 1+2 | 1+0 | 0 | Second round |
| >F2BD Mutll+ Li g11 8.12.07 | YSLGSSFGSGAG SSS[?]SRTSSSRAV VVK | 1+2 | 1+0 | 0 | Second round |
| >F2bd Mutll+ Lig 1 2007-12-22 | YS[?]GSSFGSGAG SSSF[?]RTSSSRA VVVK | 1+2 | 1+0 | 0 | Second round |
| >F2bd Mutll+ Lig 2 2007-12-22 | YSLGSSFGSGAG SSSFSRTSSSRAV VVK | 1+2 | 1+0 | 0 | Second round |
| >F2bd Mutll+ Lig 4 2007-12-22 | YSLGSSFGSGAG SSSSSRTSSSRAV [?]VK | 1+2 | 1+0 | 0 | Second round |
| >F2bd Mutll+ Lig 5 2007-12-22 | YSLGSSFGSGAG SSSF[?]RTSSSRA VVV | 1+2 | 1+0 | 0 | Second round |
| >F2bd Mutll+ Lig 7 2007-12-22 | YSLGSSFGRGAG SSSFSRRSSSRAV VVK | 1+2 | 1+0 | 0 | Second round |
| >F2bd Mutll+ Lig 8 2007-12-22 | YSLGSSFG[?]GAG SSSFSRTSSSRAV VV[?] | 1+2 | 1+0 | 0 | Second round |
| >F2bd Mutll+ Lig 11 2007-12-22 | YSLGSSFGSGAG SSSFSRTSSSRAV VVK | 1+2 | 1+0 | 0 | Second round |
| >F2bd Mutll+ Lig 14 2007-12-22 | Y[?]LGSSFGSGAG SSSFSRTSSSRAV VVK | 1+2 | 1+0 | 0 | Second round |
| Table 11 | | | | | |

| Peptide Name | Aminoacid Sequence | Mutagenesis | Selection | Selection | Rounds of Mutagenesis & Selection |
|---|---|---|---|---|---|
| | | | Moderate | Strong | |
| >Frag2binding domain | YSLGSSFGSGAG SSSFSRTSSSRAV VVK | 1 | 1 | 0 | First round |
| | | | | | |
| >0mM 1 2008-04-08 | YSLGSSFGSGAG SSSFSRTSSSRAV VVK | 1+2 | 1+2 | 0 | Second round |
| >0mM 2 2008-04-08 | YSLGSSFGSGAG SSSFSRTSS[?]RAV VVK | 1+2 | 1+2 | 0 | Second round |
| >0mM 3 2008-04-09 | YSLGSSFGSGAG SSSFSRTSSSRAV VVK | 1+2 | 1+2 | 0 | Second round |
| >0mM 5 2008-04-09 | YSLGSSFGSGAG SS[?][?][?]RTSSSRA VVVK | 1+2 | 1+2 | 0 | Second round |
| >0mM 7 2008-04-09 | YSLGSSFGSGAG SRSFSRTSSSRA VVVK | 1+2 | 1+2 | 0 | Second round |
| >0mM 8 2008-04-09 | YSLGSSFGSGAG SSS[?]SRTSSSRA VVVK | 1+2 | 1+2 | 0 | Second round |
| Table 12 | | | | | |

| Peptide Name | Aminoacid Sequence | Mutagene sis | Selection | Selection | Rounds of Mutagenesis & Selection |
|---|---|---|---|---|---|
| | | | Moderate | Strong | |
| >Frag2binding domain | YSLGSSFGSGAG SSSFSRTSSSRA VVVK | 1 | 1 | 0 | First round |
| | | | | | |
| >250mM 2 2008-04-09 | YSLGSSFGSGAG [?]SSFSRTSSSRA VVVK | 1+2 | 1+0 | 0+2 | Second round |
| >250mM 3 2008-04-09 | YSLGSSFGSGAG S[?]SFSRTSSSRA VVVK | 1+2 | 1+0 | 0+2 | Second round |
| >250mM 5 2008-04-09 | Y[?]LGSSFGSGAG SSSF[?]RTSSSRA VVVK | 1+2 | 1+0 | 0+2 | Second round |
| >250mM 6 2008-04-09 | YSLGSSFGSGAG S[?]SFSR[?]SSSRA VVVK | 1+2 | 1+0 | 0+2 | Second round |
| >250mM 7 2008-04-09 | YS[?]GSSFGSAG SSSF[?]RTSSSRA VVVK | 1+2 | 1+0 | 0+2 | Second round |
| >250mM 9 2008-04-09 | YSLGSSFGSGAG S[?]SFSRTSSSRA VVVK | 1+2 | 1+0 | 0+2 | Second round |
| >250mM 8 2008-04-09 | YSLGSSFGSGAG SSS[?]SRTSSSRA VVVK | 1+2 | 1+0 | 0+2 | Second round |
| Table 13 | | | | | |

| Peptide Name | Aminoacid Sequence | Mutagene sis | Selection | Selection | Selection | Rounds of Mutagenesis & Selection |
|---|---|---|---|---|---|---|
| | | | | | | |
| | | | Moderate | Strong | Very strong | |
| >Frag2binding domain | YSLGSSFGSGA GSSSFSRTSSSR AVVVK | 1 | 1 | 0 | 0 | First round |
| | | | | | | |
| >Hefeklon1 2008-02-13 | YSLGSSFGSGA GSSSGRTSSS RAVVVK | 1+2 | 1+0 | 0 | 0+1 | Second round |
| >Hefeklon2 2008-02-13 | YSLGSSFGSGA GSSS[?]GRTSSS RAVVVK | 1+2 | 1+0 | 0 | 0+1 | Second round |
| >Hefeklon7 2008-02-13 | YSLGSSFGSGA G[?]SS[?]SRTS[?]S RAVVVK | 1+2 | 1+0 | 0 | 0+1 | Second round |
| Table 14 | | | | | | |

## Claims

1. Method for manufacturing a modified polypeptide from a first polypeptide, said modified polypeptide exhibiting an increased binding affinity to a target molecule compared to a first polypeptide, comprising the steps of:
a) providing a first cell comprising (i) a nucleic acid molecule encoding for a first fusion polypeptide, said first fusion polypeptide comprising at least one first polypeptide and a transcriptional activation domain; and (ii) a nucleic acid molecule encoding for a second fusion polypeptide, said second fusion polypeptide comprising the target molecule or a polypeptide domain binding the target molecule and a DNA binding domain, whereby the cell further comprises a reporter gene encoding a reporter polypeptide operably linked to an upstream transcriptional regulatory sequence comprising a DNA binding site for the DNA binding domain of the second fusion polypeptide,
b) cultivating the cells of step a),
c) identifying and pooling cells expressing the reporter polypeptide,
d) isolating the nucleic acid molecules encoding for the at least one first polypeptide of the pooled cells of step c),
e) modifying the nucleic acid molecules of step d) by introducing at least one mutation thus obtaining modified nucleic acid molecules encoding for a library of modified polypeptides,
f) introducing the modified nucleic acid molecules of step e) into at least one second cell comprising a nucleic acid molecule encoding for a second fusion polypeptide, said second fusion polypeptide comprising the target molecule or a polypeptide domain binding the target molecule and a DNA binding domain, and
g) repeating steps a) to f) at least two additional times until a nucleic acid molecule encoding for a modified polypeptide is obtained and isolated in step d) exhibiting at least 20% higher binding affinity to the target molecule compared to the first polypeptide, wherein in the repeating steps a) to d) the first polypeptide is exchanged with the library of modified polypeptides of step e).

2. Method according to claim 1, **characterised in that** the nucleic acid molecule encoding for the first fusion polypeptide and/or the nucleic acid molecule encoding for the second fusion polypeptide are comprised on at least one vector.

3. Method according to claim 1 or 2, **characterized in that** the expression rate of the reporter polypeptide of the at least one cell identified in step c) is quantified.

4. Method according to any one of claims 1 to 3, **characterised in that** the target molecule is a target polypeptide.

5. Method according to claim 4, **characterized in that** the target polypeptide molecule is selected from the group of receptors, structural proteins, transport proteins and enzymes, preferably cell surface receptors, transcription factors, and enzymes such as kinases, proteinases, phosphatases, other hydrolases, or translocases.

6. Method according to any one of claims 1 to 5, **characterized in that** the nucleotide sequences encoding the at least one first polypeptide are obtained from peptide and/or polypeptide libraries and/or from cDNA, genomic DNA or Expressed Sequence Tags (ESTs) of at least one organism and/or derived from the OR-Feome of at least one organism.

7. Method according to claim 6, **characterized in that** the at least one organism is a plant or an animal, preferably a mammal.

8. Method according to any one of claims 1 to 7, **characterized in that** the at least one mutation of the nucleotide sequences of step e) is a point mutation, a deletion, an insertion, a DNA translocation, DNA shuffeling, DNA rearrangements, DNA fragmentation, DNA multimerisation or a combination thereof.

9. Method according to any one of claims 2 to 8, **characterized in that** the vector is a prokaryotic hybrid vector, preferably a bacterial hybrid vector, or a eukaryotic hybrid vector, preferably a yeast, insect or mammalian hybrid vector.

10. Method according to any one of claims 1 to 9, **characterized in that** the cell is a prokaryotic cell, preferably a bacterial cell, or a eukaryotic cell, preferably a yeast, insect or mammalian cell.

## Patentansprüche

1. Verfahren zur Herstellung eines modifizierten Polypeptids aus einem ersten Polypeptid, wobei das modifizierte Polypeptid im Vergleich zu einem ersten Polypeptid über eine erhöhte Affinität der Bindung an ein Zielmolekül verfügt, umfassend die folgenden Schritte:
a) das Bereitstellen einer ersten Zelle, umfassend (i) ein Nukleinsäuremolekül, das für ein erstes Fusionspolypeptid kodiert, wobei das erste Fusionspolypeptid mindestens ein erstes Polypeptid und eine Transkriptionsaktivierungsdomäne umfasst; und (ii) ein Nukleinsäuremolekül, das für ein zweites Fusionspolypeptid kodiert, wobei das zweite Fusionspolypeptid das Zielmolekül oder eine an das Zielmolekül bindende Polypeptiddomäne und eine DNA-bindende Domäne umfasst, wobei die Zelle des Weiteren ein Reportergen umfasst, das für ein Reporterpolypeptid kodiert, das funktionell mit einer stromaufwärts gelegenen Transkriptionsregulationssequenz verknüpft ist, die eine DNA-Bindungsstelle für die DNA-bindende Domäne des zweiten Fusionspolypeptids umfasst;
b) das Kultivieren der Zellen aus Schritt a);
c) das Identifizieren und Poolen von Zellen, die das Reporterpolypeptid exprimieren;
d) das Isolieren der Nukleinsäuremoleküle, die für das mindestens ein erstes Polypeptid der gepoolten Zellen aus Schritt c) kodieren;
e) das Modifizieren der Nukleinsäuremoleküle aus Schritt d) durch das Einbringen mindestens einer Mutation, wodurch modifizierte Nukleinsäuremoleküle erhalten werden, die für eine Bibliothek von modifizierten Polypeptiden kodieren;
f) das Einbringen der modifizierten Nukleinsäuremoleküle aus Schritt e) in mindestens eine zweite Zelle, die ein Nukleinsäuremolekül umfasst, das für ein zweites Fusionspolypeptid kodiert, wobei das zweite Fusionspolypeptid das Zielmolekül oder eine an das Zielmolekül bindende Polypeptiddomäne und eine DNA-bindende Domäne umfasst; und
g) das mindestens zweimalige Wiederholen der Schritte a) bis f) bis ein für ein modifiziertes Polypeptid kodierendes Nukleinsäuremolekül erhalten und in Schritt d) isoliert ist, das im Vergleich zu dem ersten Polypeptid über eine um mindestens 20% erhöhte Affinität der Bindung an das Zielmolekül verfügt, wobei im Verlauf des Wiederholens der Schritte a) bis d) das erste Polypeptid durch die Bibliothek der modifizierten Polypeptide aus Schritt e) ausgetauscht wird.

2. Verfahren nach Anspruch 1, **dadurch gekennzeichnet, dass** das Nukleinsäuremolekül, das für das erste Fusionspolypeptid kodiert, und/oder das Nukleinsäuremolekül, das für das zweite Fusionspolypeptid kodiert, in mindestens einem Vektor umfasst sind.

3. Verfahren nach Anspruch 1 oder 2, **dadurch gekennzeichnet, dass** die Expressionsrate des Reporterpolypeptids der mindestens einen in Schritt c) identifizierten Zelle quantifiziert wird.

4. Verfahren nach einem der Ansprüche 1 bis 3, **dadurch gekennzeichnet, dass** es sich bei dem Zielmolekül um ein Zielpolypeptid handelt.

5. Verfahren nach Anspruch 4, **dadurch gekennzeichnet, dass** das Zielpolypeptid ausgewählt ist aus der Gruppe bestehend aus Rezeptoren, Strukturproteinen, Transportproteinen und Enzymen, bevorzugt Zelloberflächenrezeptoren, Transkriptionsfaktoren und Enzymen wie z. B. Kinasen, Proteinasen, Phosphatasen oder Hydrolasen oder Translokasen.

6. Verfahren nach einem der Ansprüche 1 bis 5, **dadurch gekennzeichnet, dass** die Nukleotidsequenzen, die für das mindestens eine erste Polypeptid kodieren, aus Peptid- und/oder Polypeptidbibliotheken und/oder aus cDNA, genomischer DNA oder ESTs (Exprimierten Sequenz-Tags) erhalten werden, die von mindestens einem Organismus stammen und/oder von dem ORFeom mindestens eines Organismus abgeleitet sind.

7. Verfahren nach Anspruch 6, **dadurch gekennzeichnet, dass** es sich bei dem mindestens einen Organismus um eine Pflanze oder ein Tier, bevorzugt einen Säuger, handelt.

8. Verfahren nach einem der Ansprüche 1 bis 7, **dadurch gekennzeichnet, dass** es sich bei mindestens einer Mutation der Nukleotidsequenzen aus Schritt e) um eine Punktmutation, eine Deletion, eine Insertion, eine DNA-Translokation, DNA-Shuffling, DNA-Umgruppierungen, DNA-Fragmentierung, DNA-Multimerisierung oder eine Kombination aus diesen handelt.

9. Verfahren nach einem der Ansprüche 2 bis 8, **dadurch gekennzeichnet, dass** es sich bei dem Vektor um einen prokaryotischen Hybridvektor, bevorzugt einen bakteriellen Hybridvektor, oder einen eukaryotischen Hybridvektor, bevorzugt einen Hybridvektor aus Hefe, Insekten oder Säugern, handelt.

10. Verfahren nach einem der Ansprüche 1 bis 9, **dadurch gekennzeichnet, dass** es sich bei der Zelle um eine prokaryotische Zelle, bevorzugt eine bakterielle Zelle, oder eine eukaryotische Zelle, bevorzugt eine Hefe-, Insekten- oder Säugerzelle, handelt.

## Revendications

1. Procédé de production d'un polypeptide modifié à partir d'un premier polypeptide, ledit polypeptide modifié présentant une affinité de liaison accrue à une molécule cible comparée à un premier polypeptide, comprenant les étapes consistant en :
a) la mise à disposition d'une première cellule comprenant (i) une molécule d'acide nucléique codant pour un premier polypeptide de fusion, ledit premier polypeptide de fusion comprenant au moins un premier polypeptide et un domaine d'activation transcriptionnelle ; et (ii) une molécule d'acide nucléique codant pour un deuxième polypeptide de fusion, ledit deuxième polypeptide de fusion comprenant la molécule cible ou un domaine de polypeptide liant la molécule cible et un domaine liant l'ADN, dans lequel la cellule comprend en outre un gène reporter codant pour un polypeptide reporter lié fonctionnellement à une séquence régulatrice transcriptionnelle amont, comprenant un site de liaison à l'ADN pour le domaine de liaison à l'ADN du deuxième polypeptide de fusion,
b) la culture des cellules de l'étape a),
c) l'identification et le regroupement des cellules exprimant le polypeptide reporter,
d) l'isolement des molécules d'acide nucléique codant pour l'au moins un premier polypeptide des cellules regroupées de l'étape c),
e) la modification des molécules d'acide nucléique de l'étape d) par l'introduction d'au moins une mutation donnant ainsi les molécules d'acide nucléique modifiées codant pour une banque de polypeptides modifiés,
f) l'introduction des molécules d'acide nucléique modifiées de l'étape e) dans au moins une deuxième cellule comprenant une molécule diacide nucléique codant pour un deuxième polypeptide de fusion, ledit deuxième polypeptide de fusion comprenant la molécule cible ou un domaine de polypeptide liant la molécule cible et un domaine liant l'ADN, et
g) la répétition des étapes a) à f) au moins deux fois supplémentaires jusqu'à ce qu'une molécule d'acide nucléique codant pour un polypeptide modifié soit obtenue et isolée dans l'étape d) présentant au moins 20 % d'affinité de liaison en plus pour la molécule cible par rapport au premier polypeptide, dans lequel dans les étapes répétées a) à d), le premier polypeptide est échangé avec la banque de polypeptides modifiés de l'étape e).

2. Procédé selon la revendication 1, **caractérisé en ce que** la molécule d'acide nucléique codant pour le premier polypeptide de fusion et/ou la molécule d'acide nucléique codant pour le deuxième polypeptide de fusion sont comprises sur au moins un vecteur.

3. Procédé selon la revendication 1 ou 2, **caractérisé en ce que** le taux d'expression du polypeptide reporter de l'au moins une cellule identifiée à l'étape c) est quantifié.

4. Procédé selon l'une quelconque des revendications 1 à 3, **caractérisé en ce que** la molécule cible est un polypeptide cible.

5. Procédé selon la revendication 4, **caractérisé en ce que** la molécule du polypeptide cible est choisie dans le groupe des récepteurs, des protéines structurales, des protéines de transport et des enzymes, de préférence, des récepteurs de surface cellulaire, des facteurs de transcription et des enzymes telles que les kinases, les protéinases, les phosphatases, d'autres hydrolases ou les translocases.

6. Procédé selon l'une quelconque des revendications 1 à 5, **caractérisé en ce que** les séquences nucléotidiques codant pour l'au moins un premier polypeptide sont obtenues à partir de banques de peptides et/ou de polypeptides et/ou d'ADNc, d'ADN génomique ou de marqueurs de séquences exprimées (Expressed séquence Tag, EST) d'au moins un organisme et/ou dérivé de l'ORFéome d'au moins un organisme.

7. Procédé selon la revendication 6, **caractérisé en ce que** l'au moins un organisme est une plante ou un animal, de préférence, un mammifère.

8. Procédé selon l'une quelconque des revendications 1 à 7, **caractérisé en ce que** l'au moins une mutation des séquences nucléotidiques de l'étape e) est une mutation ponctuelle, une délétion, une insertion, une translocation d'ADN, un brassage de l'ADN, des réarrangements de l'ADN, une fragmentation de l'ADN, une multimérisation de l'ADN ou une combinaison de ceux-ci.

9. Procédé selon l'une quelconque des revendications 2 à 8, **caractérisé en ce que** le vecteur est un vecteur hybride procaryote, de préférence, un vecteur hybride bactérien ou un vecteur hybride eucaryote, de préférence, un vecteur de levure, d'insecte ou un vecteur hybride de mammifère.

10. Procédé selon l'une quelconque des revendications 1 à 9, **caractérisé en ce que** la cellule est une cellule procaryote, de préférence, une cellule bactérienne ou une cellule eucaryote, de préférence, une cellule de levure, d'insecte ou de mammifère.
